# EUROPEAN PATENT SPECIFICATION

(11) **EP 4 373 974 B1**
(45) Date of publication and mention of the grant of the patent: **13.05.2026**
(21) Application number: 22760868.4
(22) Date of filing: 20.07.2022
(51) Int. Cl.: C12Q 1/6886

(54) **BIOMARKERS FOR THERAPY RESPONSE AFTER IMMUNOTHERAPY**
BIOMARKER FÜR DAS ANSPRECHEN AUF EINE THERAPIE NACH EINER IMMUNTHERAPIE
BIOMARQUEURS POUR LA RÉPONSE THÉRAPEUTIQUE APRÈS UNE IMMUNOTHÉRAPIE

(30) Priority: 22.07.2021 EP 21187175
(43) Date of publication of application: 29.05.2024
(73) Proprietor: Asylia Diagnostics BV, 2340 Beerse (BE)
(72) Inventor: KHMELEVSKIY, Andrey, 1000 Brussel (BE)
(74) Representative: De Clercq & Partners
(86) International application number: PCT/EP2022/070304
(87) International publication number: WO 2023/001874

(56) References cited:
- EP-A1- 3 299 478
- WO-A1-2016/094377
- WO-A1-2017/013436
- WO-A1-2021/030627
- WO-A2-2020/117952
- GIDE TUBA N ET AL: "Distinct Immune Cell Populations Define Response to Anti-PD-1 Monotherapy and Anti-PD-1/Anti-CTLA-4 Combined Therapy", CANCER CELL, vol. 35, no. 2, 11 February 2019 (2019-02-11), pages 238, XP085598911, ISSN: 1535-6108, DOI: 10.1016/J.CCELL.2019.01.003
- XIONG DONGHAI ET AL: "A gene expression signature of TREM2hi macrophages and [gamma][delta] T cells predicts immunotherapy response", NATURE COMMUNICATIONS, vol. 11, no. 1, 1 December 2020 (2020-12-01), XP055880661, DOI: 10.1038/s41467-020-18546-x

## Description

### FIELD OF THE INVENTION

The invention relates to the field of diagnostics to guide cancer immunotherapy. More specifically, the invention provides methods and kits for predicting and/or determining the response of a subject to treatment with an immunotherapeutic agent.

### BACKGROUND TO THE INVENTION

Cancer is a major public health problem. In 2015, 1.3 million people died from cancer in the European Union, which equated to more than one quarter (25.4%) of the total number of deaths. Many treatments have been devised for various cancers.

Immune checkpoint inhibitors (ICls) have changed the treatment landscape for many tumor types, particularly in the metastatic setting. The development of these ICls targeting cytotoxic T-lymphocyte antigen-4 (CTLA-4) and programmed cell death-1 (PD-1/PD-L1) has significantly improved the treatment of a variety of cancers, like melanoma and non-small cell lung carcinoma (NSCLC). ICls enhance antitumor immunity by blocking the prototypical immune checkpoint receptors that exist both on immune cells and on tumor cells and exhibit negative regulation of T-cell function. Although these inhibitors can lead to remarkable responses, patients with cancer respond differently to an ICI treatment. Some patients quickly improve and are able to overcome the disease, counting for about 25% to 30% of the treated patients, but 50% of the treated patients see no durable benefits. Even more alarming, a growing number of studies shows that immunotherapy may accelerate tumor progression in a significant subset of patients ranging from 4% to 29% across multiple histologies and lead to so-called hyperprogression.

Currently available diagnostics are not efficient enough to prognostically identify which patients are going to respond to immunotherapy, such as ICI therapy, or to identify which patients will experience hyperprogressive disease or be non-responders to the ICI therapy. Notably, at best the accuracy of available biomarkers or sets of biomarkers falls below 52-58% and it does not provide much insight into the biological mechanism underlying the non-responsiveness to ICls. For example, WO2014151006 provides biomarkers for patient selection and prognosis in cancer. However, this patent application is limited to predicting the responsiveness of an individual with a disease to treatment with a PD-L1 axis binding antagonist. WO2019012147 proposes a radiomics based biomarker for detecting the presence and density of tumor infiltrating CD8 T-cells to prognose the survival and/or the treatment efficiency of cancer patients treated with immunotherapy such as anti-PD-1/PD-L1 monotherapy. US20180107786 discloses a method for generating an immune score based on tumor infiltrating lymphocytes, T-cell receptor signaling and mutation burden. WO2021030627 provides biomarkers for predicting a patient's response to checkpoint inhibitor therapy.

Accordingly, there is a need for assays capable of characterizing an immunological tumor microenvironment for companion diagnostics development and therapeutic decisions. In particular, there is a high need for biomarkers to identify responders and non-responders to immunotherapy treatment.

### SUMMARY OF THE INVENTION

The inventors have addressed the challenges for predicting and/or determining the response of a subject diagnosed with cancer to treatment with an immunotherapeutic agent by identifying biomarkers that are able to predict the response of the subject to the immunotherapeutic agent based on the RECIST response criteria. With the present invention, the inventors thus identified biomarkers that are able to predict whether a subject will respond, partially respond or not respond to treatment with an immunotherapeutic agent, more specifically with an immune checkpoint inhibitor or a combination of immune checkpoint inhibitors.

Accordingly, a first aspect of the present invention relates to methods for determining and/or predicting the response to treatment with an immunotherapeutic agent that is an immune checkpoint inhibitor or a combination of immune checkpoint inhibitors in a subject diagnosed with cancer, and wherein said methods are based on the analysis of the expression level of the biomarkers CD247, LAX1, and IKZF3 in a sample of the subject wherein the sample contains or is suspected to contain tumor cells. More specifically, the methods are based on the analysis of the RNA expression level of the protein coding genes, and wherein on the basis of said expression level it is predicted whether the subject will respond to an immunotherapeutic agent. In an embodiment, the expression level of the biomarkers CD247, LAX1 and IKZF3 is analyzed in a sample of the subject that contains or is suspected to contain tumor cells, and the expression level of these biomarkers is used to determine and/or predict the response to treatment with an immunotherapeutic agent in the subject. In still a further embodiment, the expression level of the biomarkers CD247, LAX1 and IKZF3 is analyzed in a sample of the subject that contains or is suspected to contain tumor cells, in combination with the expression level of the biomarkers CD3G and ITGAL. In still a further embodiment, the expression level of the biomarkers CD247, LAX1, IKZF3, CD3G and ITGAL is analyzed in a sample of the subject that contains or is suspected to contain tumor cells in combination with the expression level of at least 2; preferably at least 4; even more preferably all of the biomarkers CD3E, TIGIT, PIK3CD, IGHV1.3, IGHV4.4.

In some embodiments of the methods, the expression level of the analyzed biomarkers is compared to a corresponding reference value or threshold value that is characteristic of a subject with a known response to treatment with the immunotherapeutic agent.

In some embodiments, the methods of the present invention further comprise obtaining a risk score on the basis of the expression level of the analyzed biomarkers, said risk score representing the likelihood for responding to the treatment with the immunotherapeutic agent in the subject.

In some embodiments, the response is a RECIST1.1 criteria response, such as early death (ED), progressive disease (PD), stable disease (SD), partial response (PR), and complete response (CR).

In another aspect, the use of a kit in a method for determining and/or predicting the response of a subject diagnosed with cancer to an immunotherapeutic agent that is an immune checkpoint inhibitor or a combination of immune checkpoint inhibitors and according to the first aspect is provided. Said kit comprises means for measuring the expression level of the biomarkers CD247, LAX1, and IKZF3 in a sample of the subject; optionally, means for measuring the expression level of the biomarkers ITGAL andCD3G in a sample of the subject; optionally, means for measuring the expression level of the biomarkers CD3E, TIGIT, PIK3CD, IGHV1.3, IGHV4.4 in a sample of the subject; and optionally, a reference value or threshold value for the biomarkers CD247, LAX1, and IKZF3;optionaly, a reference value or threshold value for the biomarkers ITGAL and CD3G; optionally, a reference value or threshold value for the biomarkers CD3E, TIGIT, PIK3CD, IGHV1.3, and IGHV4.4.

In some embodiments, the methods of the application are further characterized in that the risk score is obtained and calculated using a pre-trained machine learning model and using the expression level of the biomarkers as input values. In particular, the risk score is produced by a machine learning model that uses the normalized expression levels of the biomarker signature as input data. More specifically, the machine learning model uses a pre-trained architecture to calculate the risk score from 0 to 1.

Further provided is a computer-implemented method for monitoring, predicting or determining the response of a subject diagnosed with cancer to treatment with an immunotherapeutic agent that is an immune checkpoint inhibitor or a combination of immune checkpoint inhibitors is provided. Said method comprising: (a) providing quantified expression levels of the biomarkers CD247, LAX1 and IKZF3 of a sample of the subject wherein the sample contains or is suspected to contain tumor cells; (b) optionally providing quantified expression levels of the biomarkers CD3G and ITGAL of a sample of the subject wherein the sample contains or is suspected to contain tumor cells; and/or providing quantified expression levels of the biomarkers CD3E, TIGIT, PIK3CD, IGHV1.3, IGHV4.4; (c) normalizing the quantified expression levels whereby normalization occurs via comparison with data obtained from corresponding assessments and expression levels from a reference set; (d) classifying whether the normalized values of step (c) exceed a predetermined threshold; (e) obtaining a risk score of said normalized value, wherein said risk score is calculated using a pre-trained machine learning model, and wherein the risk score represents the likelihood for the response to the immunotherapeutic agent by the subject.

The methods according to the present application are characterized in that the immunotherapeutic agent is an immune checkpoint inhibitor or a combination of several different immune checkpoint inhibitors. In some further preferred embodiments, the immunotherapeutic agent is selected from the group consisting of a PD-1 targeting agent, a PD-L1 targeting agent, and a CTLA-4 targeting agent, or a combination thereof. In some further preferred embodiments, a PD-1 targeting agent or a PD-L1 targeting agent is combined with a CTLA-4 targeting agent as immunotherapeutic treatment.

As outlined above, in the methods of the present application, a sample of a subject diagnosed with cancer is analyzed to quantify the expression level of one or more biomarkers; in particular one or more protein coding genes. The sample contains tumor cells or is suspected to contain tumor cells. In some embodiments, the sample contains tumor cells. The sample is preferably a tumor tissue sample or a blood sample comprising circulating tumor cells or that is suspected to comprise tumor cells, such as a biopsy sample or a liquid biopsy sample, derived from the subject; preferably the subject diagnosed with cancer. In some embodiments, the sample is a tumor tissue sample. In some embodiments, the sample is a blood sample comprising circulating tumor cells. In some embodiments, the sample is a sample of isolated circulating tumor cells. In some embodiments, said sample is a tumor tissue sample derived from cancer patients diagnosed with bladder cancer, kidney cancer, liver cancer, lung cancer, pancreatic cancer, prostate cancer, thyroid cancer, uterine cancer, ovarian cancer, colorectal cancer, breast cancer, head and neck cancer, skin cancer; even more preferably cancer patients diagnosed with skin cancer or melanoma, lung cancer such as lung adenocarcinoma, lung squamous cell carcinoma, non-small cell lung carcinoma (NSCLC). In preferred embodiments, the cancer is melanoma; preferably stage II, stage III or stage IV melanoma. In some embodiments, said sample is a blood sample comprising circulating tumor cells or a sample of isolated circulating tumor cells derived from cancer patients diagnosed with bladder cancer, kidney cancer, liver cancer, lung cancer, pancreatic cancer, prostate cancer, thyroid cancer, uterine cancer, ovarian cancer, colorectal cancer, breast cancer, head and neck cancer, skin cancer; even more preferably cancer patients diagnosed with skin cancer or melanoma, lung cancer such as lung adenocarcinoma, lung squamous cell carcinoma, or non-small cell lung carcinoma (NSCLC). In preferred embodiments, the cancer is melanoma. In some further preferred embodiments, the cancer is stage II, stage III or stage IV melanoma.

### BRIEF DESCRIPTION OF THE DRAWINGS

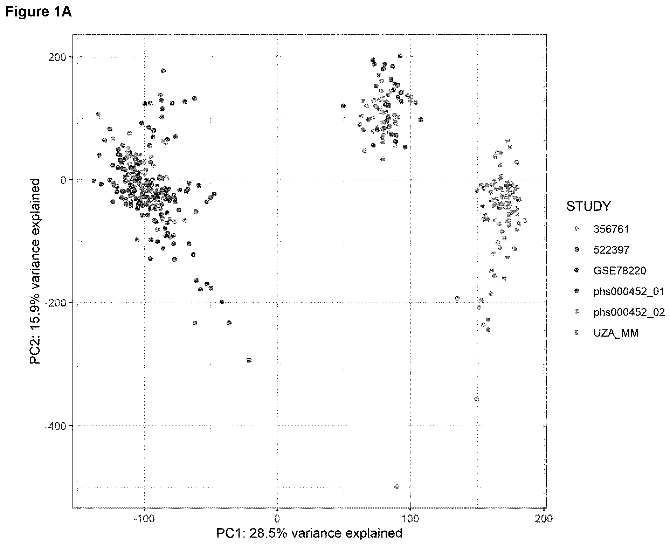

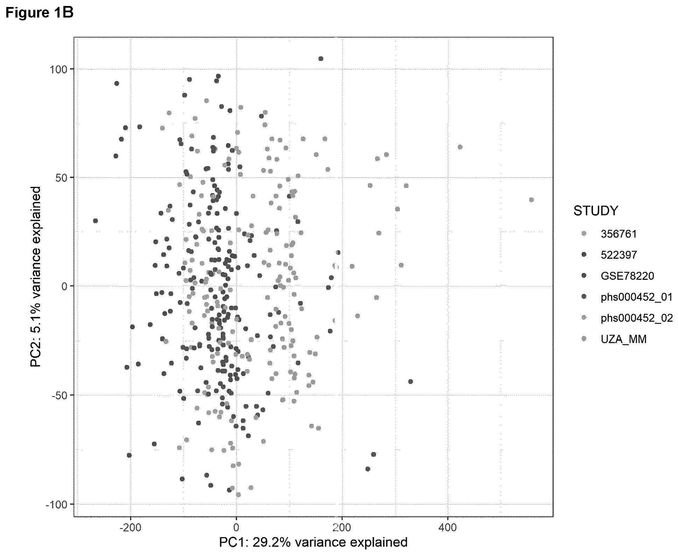

**Figure 1****. (A)** PCA analysis of the source datasets (log2 normalized rna-expression values - pre-combat). **(B)** PCA analysis of the source datasets (log2 normalized rna-expression values - post-comba).

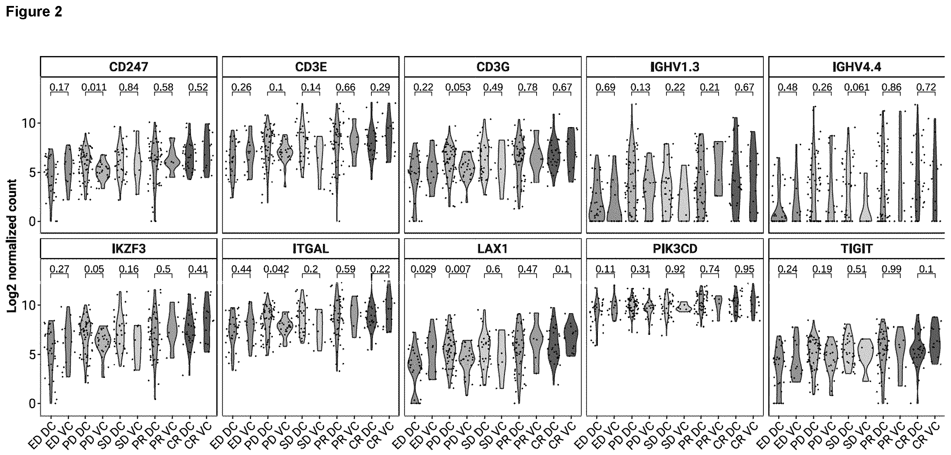

**Figure 2****.** Post-combat normalized RNA-expression levels between the discovery (DC) and validation cohorts (VC) (top 10 biomarkers based on LASSO-learner machine learning model). ED - early death; PD - progressive disease; SD - stable disease; PR - partial response; CR - complete response.

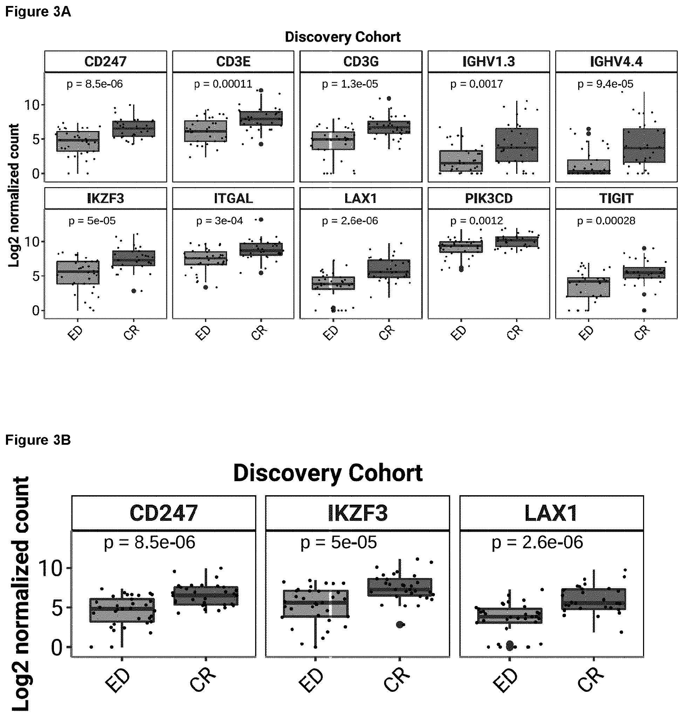

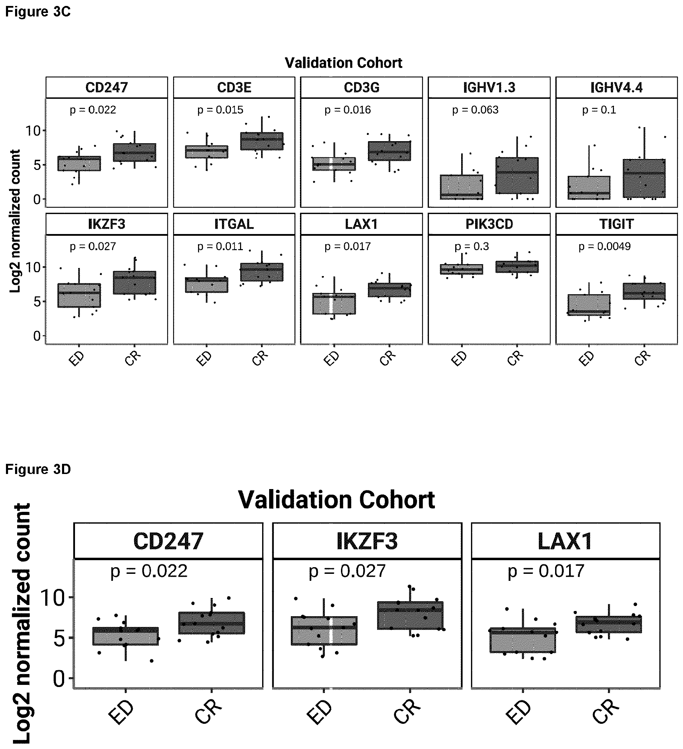

**Figure 3****. (A)** Discovery cohort: top 10 markers based on LASSO-learner machine learning model. ED - early death; CR - complete response; **(B)** Discovery cohort: top 3 markers based on LASSO-learner machine learning model. ED - early death; CR - complete response; **(C)** Validation cohort: top 10 markers identified on discovery cohort. ED - early death; CR - complete response; **(D)** Validation cohort: top 3 markers identified on discovery cohort. ED - early death; CR - complete response.

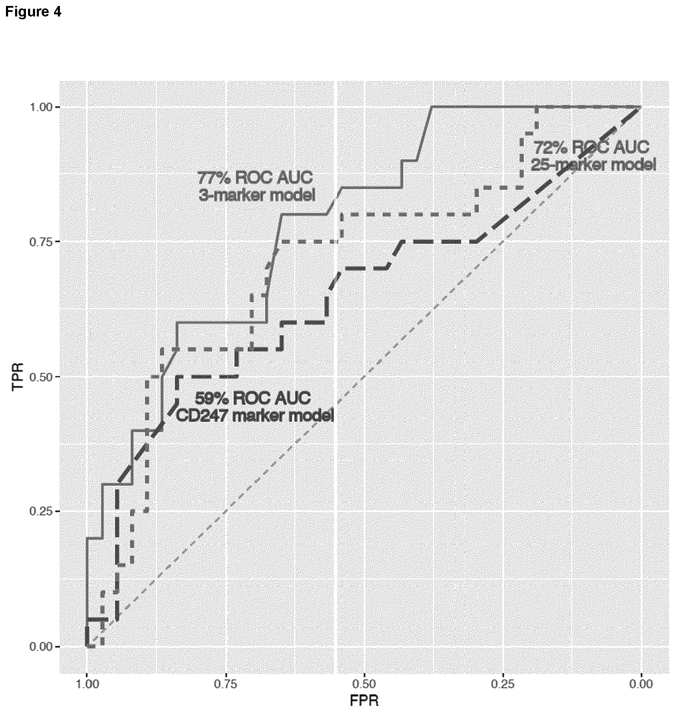

**Figure 4****.** ROC AUC curves for ED+PD vs PR+CR prediction based on random forest binary classifier. The model is trained on 75% of the discovery cohort and then applied to the validation cohort. No data mixing is done between cohorts and datasets are fully independent. The blue solid line shows ROC AUC for 3-marker model (CD247, LAX1, IKZF3); the violet dashed line shows ROC AUC for 25-marker model and the green dashed line shows ROC AUC for baseline model (CD274-only); ED - early death; CR - complete response.

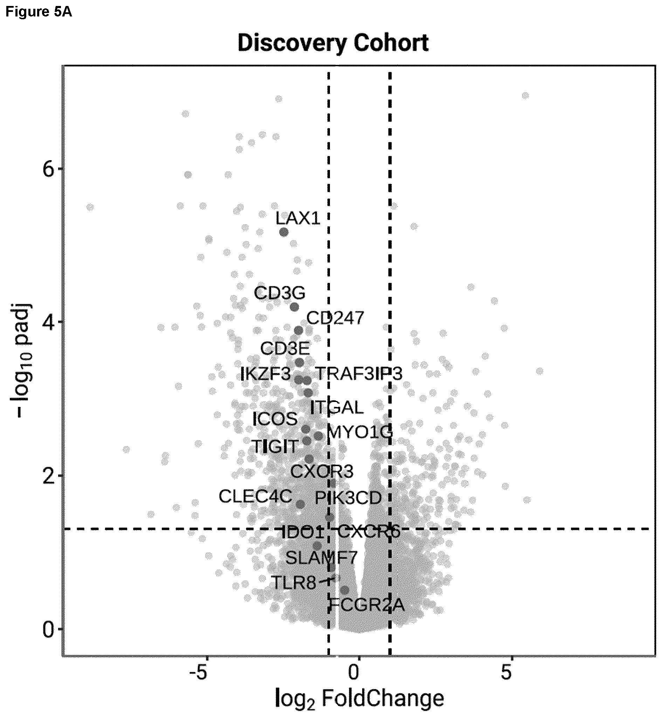

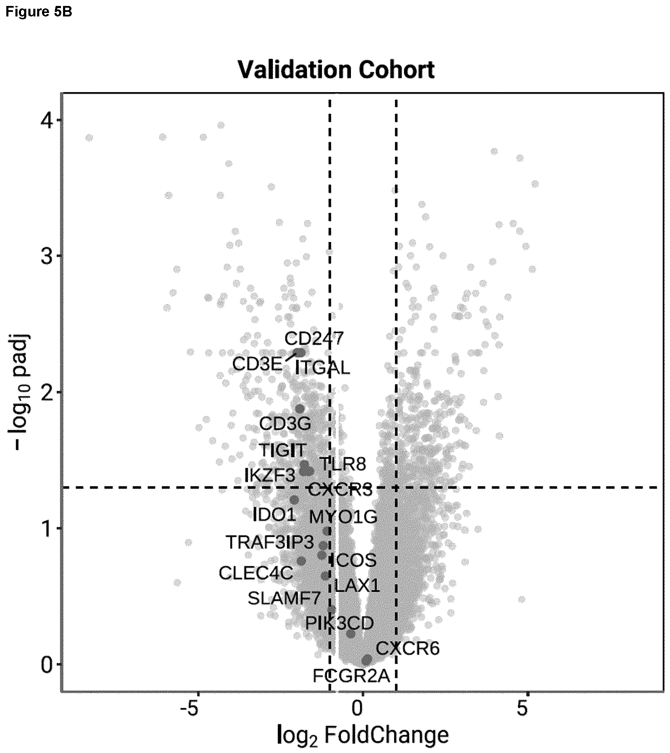

**Figure 5****. (A)** Volcano plot for DEG biomarkers between ED vs CR groups in the discovery cohort. The highlighted markers are those identified by the LASSO-learner ML model. ED - early death; CR - complete response; **(B)** Volcano plot for DEG biomarkers between ED vs CR groups in the validation cohort. The highlighted markers are those identified by the LASSO-learner ML model. ED - early death; CR - complete response.

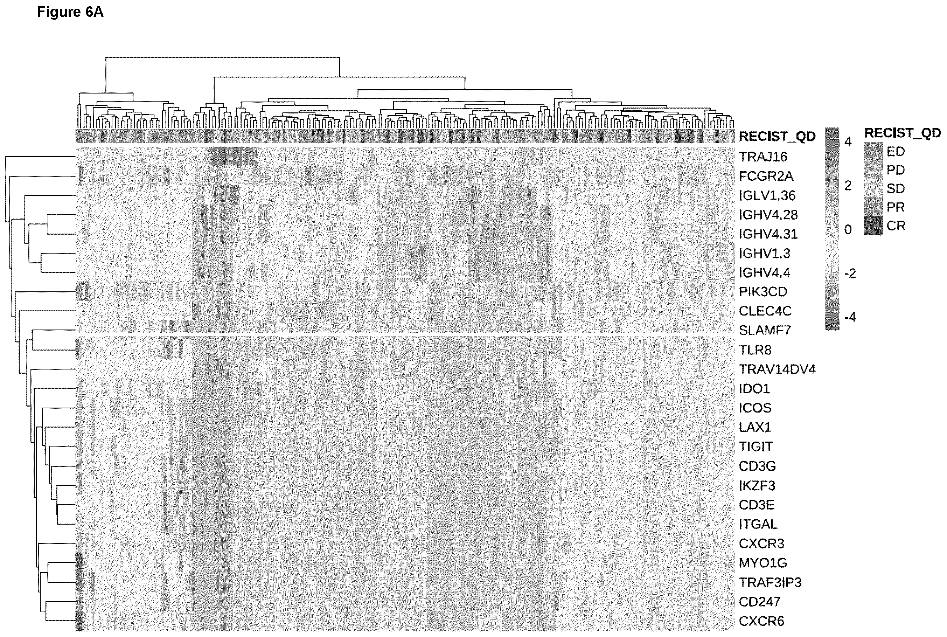

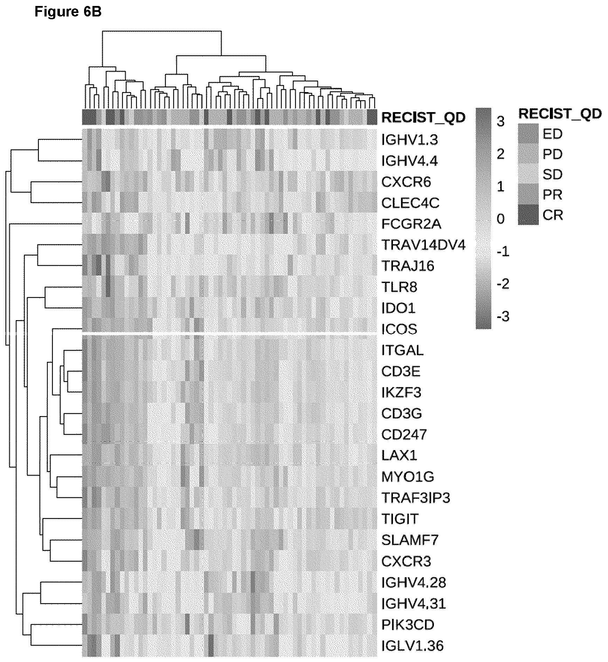

**Figure 6****. (A)** Heatmap showing scaled, log-normalized expression of 25 biomarkers for the discovery cohort. The hierarchical clustering reveals a prominent cluster of downregulated gene expression, which is enriched in ED and PD patients. **(B)** Heatmap showing scaled, log-normalized expression of 25 biomarkers for the validation cohort. The hierarchical clustering reveals a prominent cluster of downregulated gene expression, which is enriched in ED and PD patients. ED - early death; PD - progressive disease; SD - stable disease; PR - partial response; CR - complete response.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, elements, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear per se, by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

"Gene" is generally used herein to encompass a polynucleotide that encodes a gene product, e.g*.*, a nucleic acid sequence defining an open reading frame.

"Diagnosis" as used herein generally includes determination of a subject's susceptibility to a disease or disorder, determination as to whether a subject is presently affected by a disease or disorder, as well as to the prognosis of a subject affected by a disease or disorder.

The terms "individual," "subject," "host," and "patient," used interchangeably herein and refer to any mammalian subject for whom diagnosis, treatment, or therapy is desired, particularly humans.

The term "sample" or "biological sample" encompasses a variety of sample types obtained from an organism and can be used in a diagnostic or monitoring assay. The term encompasses blood and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples.

The terms "cancer", "neoplasm", "tumor", and "carcinoma", are used interchangeably herein to refer to cells which exhibit relatively autonomous growth, so that they exhibit an aberrant growth phenotype characterized by a significant loss of control of cell proliferation. In general, cells of interest for detection in the present application include immune cells in the tumor microenvironment.

The term "differentially expressed gene" is intended to encompass a polynucleotide that represents or corresponds to a gene that is differentially expressed in a tumor cell when compared with another cell of the same cell type. Such differentially expressed gene may include an open reading frame encoding a gene product (e.g., a polypeptide), as well as introns of such genes and adjacent 5' and 3' non-coding nucleotide sequences involved in the regulation of expression, up to about 20 kb beyond the coding region, but possibly further in either direction. In general, a difference in expression level associated with a decrease in expression level of at least about 25%, usually at least about 50% to 75%, more usually at least about 90% or more is indicative of a differentially expressed gene of interest, i.e., a gene that is downregulated in the test sample relative to a control sample. Furthermore, a difference in expression level associated with an increase in expression of at least about 25%, usually at least about 50% to 75%, more usually at least about 90% and may be at least about 1,5-fold, usually at least about 2-fold to about 10-fold, and may be about 100-fold to about 1,000-fold increase relative to a control sample is indicative of a differentially expressed gene of interest, i.e., an overexpressed or upregulated gene.

"Differentially expressed polynucleotide" as used herein means a nucleic acid molecule (RNA or DNA) comprising a sequence that represents a differentially expressed gene, e.g., the differentially expressed polynucleotide comprises a sequence (e.g., an open reading frame encoding a gene product) that uniquely identifies a differentially expressed gene so that detection of the differentially expressed polynucleotide in a sample is correlated with the presence of a differentially expressed gene or gene product of a differentially expressed gene in a sample. "Differentially expressed polynucleotides" is also meant to encompass fragments of the disclosed polynucleotides, e.g., fragments retaining biological activity, as well as nucleic acids that are homologous, substantially similar, or substantially identical (e.g., having about 90% sequence identity) to the disclosed polynucleotides.

The term "microenvironment," as used herein, may refer to the tumor microenvironment as a whole or to an individual subset of cells within the microenvironment. Immune cells found within the tumor microenvironment are macrophages, monocytes, mast cells, helper T cells, cytotoxic T cells, regulatory T cells, natural killer cells, myeloid-derived suppressor cells, regulatory B cells, neutrophils, dendritic cells, and fibroblasts. The tumor microenvironment is generally defined as a complex mixture of cells, soluble factors, signaling molecules, extracellular matrices, and mechanical cues that promote neoplastic transformation, support tumor growth and invasion, and protect the tumor from host immunity.

As used herein, the terms "marker(s)", "biomarker(s)" refer to a gene or genes or a protein, polypeptide, or peptide expressed by the gene or genes whose expression level, alone or in combination with other genes, is correlated with the response to an immunotherapeutic treatment. The correlation can relate to either an increased or decreased expression of the gene (e.g. increased or decreased levels of mRNA or the peptide encoded by the gene).

As used herein, the term "in vitro" refers to an artificial environment and to processes or reactions that occur within an artificial environment. In vitro environments can consist of, but are not limited to, test tubes and cell culture. The term "in vivo" refers to the natural environment (e.g., an animal or a cell) and to processes or reaction that occur within a natural environment. The term "in silico" refer to artificial environment, wherein a procedure is performed or modelled using a computer system, thereby partly or entirely avoiding the need of physically manipulating the data (e.g. genes, polynucleotides, proteins, ...).

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, the appearance of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

With the present invention, the inventors identified biomarkers for predicting and/or determining the response to treatment with an immunotherapeutic agent in a subject. More specifically, the inventors show that based on the expression level of the 3 biomarkers s CD247, LAX1, and IKZF3, the response to immunotherapy of a subject diagnosed with cancer can be determined or predicted. The inventors found that based on the expression level of a set of only 3 biomarkers, CD247, LAX1 and IKZF3, the response to immunotherapy can be predicted. This set of 3 biomarkers can optionally be extended with the biomarkers CD3G and ITGAL. Even further, the set of 5 biomarkers being CD247, LAX1, IKZF3, CD3G and ITGAL, can further be extended with the biomarkers CD3E, TIGIT, PIK3CD, IGHV1.3, IGHV4.4, thereby forming a 10-biomarker signature for response prediction to immunotherapeutic treatment.

Immunotherapy using immune checkpoint inhibitors has changed the treatment landscape for many tumor types, particularly in the metastatic setting. Though, while meaningful, durable responses are achieved in some patients, a majority of patients only partially respond, or do not respond, or even worrisome, some patients develop hyperprogressive disease, a phenomenon clinically defines as an unexpected acceleration of the tumor kinetics measured on imaging with dynamic parameters. Hyperprogressive disease (HPD) following treatment with immunotherapy such as immune checkpoint inhibitors (ICls) often leads to patient death within 24-65 days after the ICI treatment (Ferrara et al., 2020a).

To date, the search for biomarkers to predict the response to immunotherapeutic treatment, and even to predict whether a patient would develop HPD, has been challenging by the dynamic interplay between the ICls and the immune microenvironment and the heterogeneity of the immune milieu in different tumor types (Davis and Patel, 2019). With the present application, the inventors identified several biomarkers that can predict and/or determine the response of a subject to an immunotherapeutic treatment.

In a first aspect, the current application provides a method for determining and/or predicting the response to treatment with an immunotherapeutic agent that is an immune checkpoint inhibitor or a combination of immune checkpoint inhibitors in a subject diagnosed with cancer, based on the expression analysis of several biomarkers. More in particular, the expression level of 3 biomarkers CD247, LAX1, and IKZF3, will be assessed in a sample of the subject that is diagnosed with cancer and used in a method for determining and/or predicting the response to treatment with an immunotherapeutic agent.

Thus a method for determining and/or predicting the response or resistance to treatment with an immunotherapeutic agent that is an immune checkpoint inhibitor or a combination of immune checkpoint inhibitors in a subject diagnosed with cancer is provided. Said method comprises analyzing the expression level of the biomarkers CD247, LAX1 and IKZF3 in a sample of the subject wherein the sample contains or is suspected to contain tumor cells. In a further embodiment, said method further comprises analyzing the expression level of the biomarkers CD3G and ITGAL in a sample of the subject wherein the sample contains or is suspected to contain tumor cells. In still a further embodiment, said method further also comprises analysis of the expression level of at least 2, at least 3, at least 4, or preferably all of the biomarkers CD3E, TIGIT, PIK3CD, IGHV1.3, IGHV4.4 in a sample of the subject wherein the sample contains or is suspected to contain tumor cells. Thus, a method for determining and/or predicting the response to treatment with an immunotherapeutic agent that is an immune checkpoint inhibitor or a combination of immune checkpoint inhibitors in a subject is provided, said method comprising analyzing the expression level of the biomarkers CD247, LAX1 and IKZF3 in a sample of the subject wherein the sample contains or is suspected to contain tumor cells. In some embodiments, a method for determining and/or predicting the response to treatment with an immunotherapeutic agent that is an immune checkpoint inhibitor or a combination of immune checkpoint inhibitors in a subject is provided, said method comprising analyzing the expression level of the biomarkers CD247, LAX1, IKZF3, CD3G and ITGAL in a sample of the subject wherein the sample contains or is suspected to contain tumor cells. In some embodiments, a method for determining and/or predicting the response to treatment with an immunotherapeutic agent that is an immune checkpoint inhibitor or a combination of immune checkpoint inhibitors in a subject is provided, said method comprising analyzing the expression level of the biomarkers CD247, LAX1, IKZF3, CD3G, ITGAL, CD3E, TIGIT, PIK3CD, IGHV1.3 and IGHV4.4 in a sample of the subject wherein the sample contains or is suspected to contain tumor cells. In some embodiments, any of these methods can further be supplemented with the analysis of the expression level of one or more of the biomarkers selected from the list consisting of CXCR6, SLAMF7, MYO1G, ICOS, CLEC4C, CXCR3, TLR8, FCGR2A, IDO1, TRAF3IP3, IGLV1.36, TRAV14DV4, TRAJ16, IGHV4.28, IGHV4.31 in the sample of the subject.

A method for determining and/or predicting the response to treatment with an immunotherapeutic agent that is an immune checkpoint inhibitor or a combination of immune checkpoint inhibitors in a subject is thus provided, said method comprising the analysis of the expression level of the biomarkers CD247, LAX1 and IKZF3 in a sample of the subject wherein the sample contains or is suspected to contain tumor cells.

In an embodiment, a method for determining and/or predicting the response to treatment with an immunotherapeutic agent that is an immune checkpoint inhibitor or a combination of immune checkpoint inhibitors in a subject diagnosed with cancer is thus provided, said method comprising the analysis of the expression level of the biomarkers CD247, LAX1, IKZF3, CD3G and ITGAL in a sample of the subject wherein the sample contains or is suspected to contain tumor cells.

In still another embodiment, a method for determining and/or predicting the response to treatment with an immunotherapeutic agent that is an immune checkpoint inhibitor or a combination of immune checkpoint inhibitors in a subject diagnosed with cancer is thus provided, said method comprising the analysis of the expression level of the biomarkers CD247, LAX1, IKZF3, CD3G and ITGAL in a sample of the subject wherein the sample contains or is suspected to contain tumor cells, in combination with the expression level of one or more of the biomarkers CD3E, TIGIT, PIK3CD, IGHV1.3, IGHV4.4; preferably in combination with the expression level of at least 2, at least 3 or at least 4 of the biomarkers CD3E, TIGIT, PIK3CD, IGHV1.3, IGHV4.4; even more preferably, in combination with the expression level of all of the biomarkers CD3E, TIGIT, PIK3CD, IGHV1.3, IGHV4.4.

In still a further embodiment, a method for determining and/or predicting the response to treatment with an immunotherapeutic agent that is an immune checkpoint inhibitor or a combination of immune checkpoint inhibitors in a subject diagnosed with cancer is thus provided, said method comprising the analysis of the expression level of the biomarkers CD247, LAX1, IKZF3, CD3G, ITGAL, CD3E, TIGIT, PIK3CD, IGHV1.3, IGHV4.4 in a sample of the subject wherein the sample contains or is suspected to contain tumor cells.

In another embodiment, the present application provides a method for determining and/or predicting the response to treatment with an immunotherapeutic agent that is an immune checkpoint inhibitor or a combination of immune checkpoint inhibitors in a subject, said method comprising analyzing in a sample that contains or is suspected to contain tumor cells the expression level of the biomarkers CD247, LAX1, IKZF3, CD3G, ITGAL, CD3E, TIGIT, PIK3CD, IGHV1.3, IGHV4.4 in combination with one or more of the biomarkers selected from the list consisting of CXCR6, SLAMF7, MYO1G, ICOS, CLEC4C, CXCR3, TLR8, FCGR2A, IDO1, TRAF3IP3, IGLV1.36, TRAV14DV4, TRAJ16, IGHV4.28, IGHV4.31.

In some embodiments, the expression level of the biomarkers is compared to a corresponding reference value or threshold value that is characteristic of a subject with a known response to treatment with an immunotherapeutic agent, in particular an immune checkpoint inhibitor.

In some embodiments, on the basis of one of said expression analyses, a risk score is obtained wherein said risk score represents the likelihood for the response to the immunotherapeutic agent. In some further embodiments, the risk score is compared with a threshold score that is calculated based on the expression analysis of the biomarkers in a sample obtained from a subject with a known response to treatment with an immunotherapeutic agent.

The list of biomarkers, preferably protein coding genes, including CD247, LAX1, IKZF3, ITGAL, CD3G, CD3E, CXCR6, SLAMF7, MYO1G, TIGIT, ICOS, CLEC4C, CXCR3, TLR8, FCGR2A, IDO1, TRAF3IP3, PIK3CD, IGLV1.36, TRAV14DV4, TRAJ16, IGHV1.3, IGHV4.28, IGHV4.31, IGHV4.4, thus comprises the predictive biomarkers of one aspect of the current application. Preferably, at least 3; at least 5; at least 10, or even all of these predictive biomarkers are used for determining whether and how a subject is responding to treatment with an immunotherapeutic agent.

In particular embodiments, the methods as taught herein may comprise comparing the biomarker expression levels to one or more corresponding reference values or threshold values that are characteristic of a subject with a known response to treatment with an immunotherapeutic agent, in particular immune checkpoint inhibitor. Said reference or threshold value may represent the expression level of the one or more biomarkers of a subject with a known prognosis after treatment with the immunotherapeutic agent. Said known prognosis can be a response to the treatment, a partial response to the treatment, no response to the treatment, or even the development of hyperprogressive disease in response to the treatment. In particular embodiments, said known prognosis can be any of the Response Evaluation Criteria in Solid Tumours 1.1 (RECIST 1.1; (Eisenhauer et al., 2019) response criteria selected progressive disease (PD), stable disease (SD), partial response (PR), and complete response (CR). Progressive disease (PD) is additionally separated into early death (ED) and non-early death cohorts. ED is defined as progression free survival (PFS) < 90 days and overall survival (OS) < 180 days (Ferrara et al., 2020b). In other embodiments, said reference or threshold value may represent the expression level of the one or more biomarkers of a healthy subject. Said comparison may generally include any means to determine the presence or absence of at least one difference and optionally of the size of such difference between values or profiles being compared. A comparison may include a visual inspection, an arithmetical or statistical comparison of measurements. Such statistical comparisons include, but are not limited to, applying an algorithm. If the values or biomarker expression profiles comprise at least one standard, the comparison to determine a difference in said values or biomarker expression profiles may also include measurements of these standard, such that measurement of the biomarker are correlated to measurements of the internal standards.

Reference values or threshold values for the expression level of any of the biomarker may be established according to known procedures previously employed for other biomarkers.

For example, a reference value of the expression level of a biomarker for determining whether a subject is predisposed of responding to an immunotherapeutic treatment as taught herein may be established by determining the quantity or expression of said biomarker in a sample(s) from one individual or from a population (e.g., group) of individuals characterized by a known response to treatment with the immunotherapeutic agent. Such population may comprise without limitation ≥2, ≥10, ≥100, or even several hundred of individuals or more.

In an embodiment, reference value(s) or threshold value(s) as intended herein may convey absolute quantities of the biomarker as intended herein. In another embodiment, the quantity of the biomarker in a sample from a test subject may be determined directly relative to the reference value (e.g., in terms of increase or decrease, or fold-increase or fold-decrease). Advantageously, this may allow the comparison of the quantity or expression level of the biomarker in the sample from the subject with the reference value (in other words to measure the relative quantity of the biomarker in the sample from the subject vis-à-vis the reference value) without the need first to determine the respective absolute quantities of the biomarker.

As explained, the present methods, uses, or products may involve finding a deviation or no deviation between the expression level of the one or more biomarkers in a sample of the subject as taught herein and a given reference value or threshold value.

A "deviation" of a first value from a second value or a "difference" between a first value and a second value may generally encompass any direction (e.g., increase: first value > second value; or decrease: first value < second value) and any extent of alteration.

For example, a deviation or a difference may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation or a difference may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation or a difference may refer to a statistically significant observed alteration. For example, a deviation or a difference may refer to an observed alteration which falls outside of error margins of reference values in a given population (as expressed, for example, by standard deviation or standard error, or by a predetermined multiple thereof, e.g., ±1xSD or ±2xSD or ±3xSD, or ±1xSE or ±2xSE or ±3xSE). Deviation or a difference may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation or a difference may be concluded if an observed alteration is beyond a given threshold or cut-off. Such threshold or cut-off may be selected as generally known in the art to provide for a chosen accuracy, sensitivity and/or specificity of the prediction methods, e.g., accuracy, sensitivity and/or specificity of at least 50%, or at least 60%, or at least 70%, or at least 80%, or at least 85%, or at least 90%, or at least 95%.

For example, receiver-operating characteristic (ROC) curve analysis can be used to select an optimal threshold or cut-off value of the quantity of a given biomarker for clinical use of the present diagnostic tests, based on acceptable global accuracy, sensitivity and/or specificity, or related performance measures which are well-known *per se,* such as positive predictive value (PPV), negative predictive value (NPV), positive likelihood ratio (LR+), negative likelihood ratio (LR-), Youden index, or similar.

For example, an optimal threshold or cut-off value may be selected for each individual biomarker as a local extremum of the receiver operating characteristic (ROC) curve, i.e. a point of local maximum distance to the diagonal line, as described in Robin X., PanelomiX: a threshold-based algorithm to create panels of biomarkers, 2013, Translational Proteomics, 1(1):57-64.

The person skilled in the art will understand that it is not relevant to give an exact threshold or cut-off value. A relevant threshold or cut-off value can be obtained by correlating the sensitivity and specificity and the sensitivity/specificity for any threshold or cut-off value.

It is to the diagnostic engineers to determine which level of positive predictive value/negative predictive value/sensitivity/specificity is desirable and how much loss in positive or negative predictive value is tolerable. The chosen threshold or cut-off level could be dependent on other diagnostic parameters used in combination with the present method by the diagnostic engineers.

In some embodiments, in the methods, uses or products as taught herein, a risk score will be generated. The risk scores that are generated represent the likelihood for responding to immunotherapeutic treatment in the subject. In some embodiments, said risk score ranges from 0 to 1 and represents the likelihood for responding to immunotherapeutic treatment in the subject, with 1 being 100% likelihood for responding to treatment with an immunotherapeutic agent in the subject, and 0 being 0% likelihood for responding to treatment with an immunotherapeutic agent.

In some embodiments, the likelihood values are produced by a machine learning model that uses normalized expression levels of one of the biomarker signatures as input data. In a further embodiment, the machine learning model is a pre-trained machine learning model that uses a pre-trained architecture to calculate the risk score from 0 to 1. This pre-trained architecture is trained and updated based on the pool of retrospective and prospective samples with already known annotation towards the response to immunotherapeutic treatment, in particular this pre-trained architecture is trained and updated based on the pool of reference values or threshold values obtained from samples of subjects with already known annotation towards the response to immunotherapeutic treatment.

In some embodiments, the machine learning model is selected from (1) linear mixed-effects model with random effect, (2) differential expression analysis, (3) random forest machine learning model, (4) gradient boosting machine model and (5) novel deep regression model based pre-trained architectures such as VGG-16 and ResNet-50 architectures. In some embodiments, a combination of machine learning models can be applied wherein each model is run independently on each dataset and then collectively on aggregated data to ensure that the signature replicates well on the independent trials. In some further embodiments, gene and label bootstrapping and independent cohort comparisons can be applied to exclude the probability of random fit of these models and to ensure that the one or more biomarkers are unlikely to appear in the data by chance alone.

In a particularly preferred embodiment, the analysis, in particular the biomarker expression analysis and calculation of the risk score, is thus trained and updated using machine learning, for example using the gradient boosting machine (GBM) classification methodology to learn the specific pattern of biomarker expression features responsible for the subject's response to treatment with the immunotherapeutic agent.

According to some embodiments, expression levels of the one or more biomarkers selected from CD247, LAX1, IKZF3, ITGAL, CD3G, CD3E, CXCR6, SLAMF7, MYO1G, TIGIT, ICOS, CLEC4C, CXCR3, TLR8, FCGR2A, IDO1, TRAF3IP3, PIK3CD, IGLV1.36, TRAV14DV4, TRAJ16, IGHV1.3, IGHV4.28, IGHV4.31, IGHV4.4 are used in the gradient boosting machine (GBM) classification methodology to learn the specific pattern of said biomarker expression features responsible for the subject's response to one or more immunotherapy. The response variable in those GBM models is further optimized for RECIST 1.1 criteria selected from early death (ED), progressive disease (PD), partial response (PR) and complete response (CR), and response to immunotherapy. In a particular embodiment, the expression levels of the biomarkers CD247, LAX1 and IKZF3 are used in the gradient boosting machine (GBM) classification methodology to learn the specific pattern of the biomarker expression features responsible for the subject's response to immunotherapy. In other embodiments, the expression levels of the biomarkers CD247, LAX1, IKZF3, CD3G and ITGAL are used in the gradient boosting machine (GBM) classification methodology to learn the specific pattern of the biomarker expression features responsible for the subject's response to immunotherapy. In still a further embodiment, the expression levels of the biomarkers CD247, LAX1, IKZF3, CD3G, ITGAL, in combination with at least 2, at least 3, at least 4 or preferably all of the biomarkers CD3E, TIGIT, PIK3CD, IGHV1.3, IGHV4.4 are used in the gradient boosting machine (GBM) classification methodology to learn the specific pattern of the biomarker expression features responsible for the subject's response to immunotherapy.

In some embodiments, the methods of the current invention, wherein a risk score is obtained, are further supplemented with the use of other cancer diagnostics known by a person skilled in the art.

The present methods, uses and products are thus intended to predict and/or determine a subject's response to treatment with an immunotherapeutic agent. Said subject's response can be determined based on the RECIST 1.1. response criteria and can be selected from early death (ED), progressive disease (PD), stable disease (SD), partial response (PR) and complete response (CR) (https://recist.eortc.org/recist-1-1-2/; Eisenhauer et al., 2009).

The methods, kits, or uses as taught herein, are typically characterized by the expression analysis of one or more biomarkers in a sample of a subject wherein the sample contains or is suspected to contain tumor cells. As used herein, a "biomarker" is widespread in the art and may broadly denote a biological molecule and/or detectable portion thereof whose qualitative and/or quantitative evaluation in a subject is predictive or informative (e.g., predictive, diagnostic and/or prognostic) with respect to one or more aspects of the subject's phenotype and/or genotype, such as, for example, with respect to the status of the subject as to a given disease or condition.

In preferred embodiments, the biomarkers as taught herein are protein coding genes and the expression level of said protein coding genes is evaluated. In even more preferred embodiments, RNA-based expression levels of the one or more protein coding genes are evaluated. The expression level of the protein coding genes disclosed herein may be assessed by any method known in the art suitable for determination of specific gene expression levels in a sample. Such methods are well-known and routinely implemented in the art. Gene expression analysis can for example be performed by reverse transcriptase real-time quantitative PCR, gene expression arrays, TruSeq gene expression analysis, in-situ hybridization, dye sequencing, pyrosequencing, CRISPR-Cas-based or any other form of transcriptome sequencing (total RNA-Seq, mRNA-Seq, gene expression profiling).

Additionally, a customized version of TruSeq Targeted RNA expression Kit is used for measuring the expression levels of a plurality of genes as listed herein. TruSeq Targeted RNA Expression Kit enables highly customizable mid- to high-plex gene expression profiling studies which allow defining panels of 12-1,000 assays to target individual exons, isoforms, splice junctions, coding SNPs (cSNPs), gene fusions, and non-coding RNA transcripts, plus multiplex up to 384 samples.

Also, a customized version of TruSeq Custom Amplicon Kit Dx can be used for the qualitative and/or quantitative assessment of a plurality of genes as listed herein. TruSeq Custom Amplicon Kit Dx is an FDA-approved regulated and CE-IVD-marked amplicon sequencing kit that enables clinical labs to develop their own next-generation sequencing (NGS) assays for use on the FDA-regulated and CE-IVD-marked MiSeqDx and NextSeq 550dx instruments.

In certain other embodiments, a biomarker as taught herein, may be peptide-, polypeptide- and/or protein-based. The reference to any marker, including any gene, peptide, polypeptide, protein, corresponds to the marker, gene, peptide, polypeptide, protein, commonly known under the respective designations in the art. The terms encompass such markers, genes, peptides, polypeptides, proteins of any organism where found, and particularly of animals, preferably warm-blooded animals, more preferably vertebrates, yet more preferably mammals, including humans and non-human mammals, still more preferably of humans. The terms particularly encompass such markers, genes, peptides, polypeptides, proteins with a native sequence, i.e., ones of which the primary sequence is the same as that of the markers, genes, peptides, polypeptides, proteins found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to genetic alterations, or post-transcriptional or posttranslational modifications. Any such variants or isoforms of markers, genes, peptides, polypeptides, proteins are intended herein. Accordingly, all sequences of markers, genes, peptides, polypeptides, or proteins found in or derived from nature are considered "native". The terms encompass the markers, genes, peptides, polypeptides, or proteins when forming a part of a living organism, organ, tissue or cell, when forming a part of a biological sample, as well as when at least partly isolated from such sources. The terms also encompass markers, genes, peptides, polypeptides, or proteins when produced by recombinant or synthetic means.

In certain embodiments, the biomarkers as taught herein, may be a human biomarker, such as in particular any of the biomarkers selected from CD247, LAX1, IKZF3, ITGAL, CD3G, CD3E, CXCR6, SLAMF7, MYO1G, TIGIT, ICOS, CLEC4C, CXCR3, TLR8, FCGR2A, IDO1, TRAF3IP3, PIK3CD, IGLV1.36, TRAV14DV4, TRAJ16, IGHV1.3, IGHV4.28, IGHV4.31, and IGHV4.4.

The reference to "CD247" denotes the CD247 markers, peptides, polypeptide, protein, or nucleic acid, as commonly known under said designation in the art. By means of additional guidance, CD247 is also known as "CD3-zeta", "CD3H", "CD3Q", "CD3Z", "IMD25", "T3Z", or "TCRZ". The terms denote CD247 nucleic acids as well as CD247 peptides, polypeptides and proteins as apparent from the context.

By means of an example, human CD247 mRNA is annotated under NCBI Genbank (http://www.ncbi.nlm.nih.gov/) accession numbers NM_000734.4 ("isoform 2 precursor"), NM_198053.3 ("isoform 1 precursor"), XM_011510145.2 ("isoform X2"), XM_011510144.3 ("isoform X1"), NM_001378516.1 ("isoform 4 precursor"), NM_001378515.1 ("isoform X3 precursor").

Nucleotides 65 (start codon) to 556 (stop codon) of NM_000734.4 constitute the CD247 coding sequence (CDS). By means of an example, nucleotides 65 to 556 of NM_00734.4 are reproduced below:

ATGAAGTGGAAGGCGCTTTTCACCGCGGCCATCCTGCAGGCACAGTTGCCGATTACAGA
GGCACAGAGCTTTGGCCTGCTGGATCCCAAACTCTGCTACCTGCTGGATGGAATCCTCTT
CATCTATGGTGTCATTCTCACTGCCTTGTTCCTGAGAGTGAAGTTCAGCAGGAGCGCAGA
CGCCCCCGCGTACCAGCAGGGCCAGAACCAGCTCTATAACGAGCTCAATCTAGGACGAA
GAGAGGAGTACGATGTTTTGGACAAGAGACGTGGCCGGGACCCTGAGATGGGGGGAAA
GCCGAGAAGGAAGAACCCTCAGGAAGGCCTGTACAATGAACTGCAGAAAGATAAGATGG
CGGAGGCCTACAGTGAGATTGGGATGAAAGGCGAGCGCCGGAGGGGCAAGGGGCACG
ATGGCCTTTACCAGGGTCTCAGTACAGCCACCAAGGACACCTACGACGCCCTTCACATG
CAGGCCCTGCCCCCTCGCTAA (SEQ ID NO: 1)

By means of an example, human CD247 protein sequence is annotated under NCBI Genbank accession numbers NP_000725.1, NP_932170.1, XP_011508447.1, XP_011508446.1, NP_001365445.1, NP_001365444.1.

As an example, amino acid sequence of NP_000725.1 is further reproduced below:

MKWKALFTAAILQAQLPITEAQSFGLLDPKLCYLLDGILFIYGVILTALFLRVKFSRSADAPAYQ
QGQNQLYNELNLGRREEYDVLDKRRGRDPEMGGKPRRKNPQEGLYNELQKDKMAEAYSEI
GMKGERRRGKGHDGLYQGLSTATKDTYDALHMQALPPR (SEQ ID NO: 2)

By means of an example, the human CD247 gene is annotated under NCBI Genbank Gene ID 919.

The reference to "LAX1" ("lymphocyte transmembrane adaptor 1") denotes the LAX1 markers, peptides, polypeptide, protein, or nucleic acid, as commonly known under said designation in the art. By means of additional guidance, LAX1 is also known as "LAX". The terms denote LAX1 nucleic acids as well as LAX1 peptides, polypeptides and proteins as apparent from the context. By means of an example, human LAX1 mRNA is annotated under NCBI Genbank (http://www.ncbi.nlm.nih.gov/) accession numbers NM_017773.4 ("isoform a"), NM_006711397.4 ("isoform X1"), NM_001136190.2 ("isoform b"), or NM_001282878.1 ("isoform c").

Nucleotides 384 (start codon) to 1580 (stop codon) of NM_017773.4 constitute the LAX1 coding sequence (CDS). By means of an example, nucleotides 384 to 1580 of NM_017773.4 are reproduced below:

ATGGATGGTGTCACTCCAACCCTTTCGACAATCAGAGGGAGGACCTTGGAGTCCAGCAC
TCTGCATGTGACTCCCCGCAGCCTGGACAGAAATAAAGACCAGATCACCAACATCTTTTC
CGGGTTTGCGGGACTCCTCGCCATCCTCCTGGTCGTTGCGGTTTTCTGCATCTTGTGGA
ATTGGAATAAACGGAAGAAGCGACAAGTTCCTTACCTCCGAGTTACCGTCATGCCCTTGC
TGACTTTGCCACAAACCAGACAAAGAGCCAAAAATATTTATGACATCTTGCCTTGGCGAC
AGGAAGACCTGGGGAGACATGAGTCGAGGAGTATGCGCATTTTCAGTACTGAGAGCCTC
CTCTCCAGAAATTCTGAGAGCCCGGAGCATGTGCCCTCCCAAGCAGGCAATGCCTTCCA
GGAGCATACAGCCCACATCCATGCCACAGAGTACGCGGTGGGTATCTATGACAACGCCA
TGGTCCCCCAGATGTGTGGGAACCTCACTCCCTCGGCACACTGCATCAATGTCAGAGCT
TCCAGAGACTGCGCAAGCATTTCTTCAGAGGATTCGCATGATTATGTCAATGTCCCCACA
GCAGAAGAGATTGCTGAGACTCTAGCTTCTACCAAAAGCCCTTCCAGAAATCTCTTTGTT
CTTCCCAGTACCCAGAAGCTGGAGTTTACTGAGGAAAGAGATGAGGGCTGTGGAGATGC
TGGTGACTGCACCAGTTTGTATTCTCCAGGAGCTGAGGACAGTGATTCACTCAGCAATGG
AGAAGGTTCTTCTCAGATCTCAAATGACTATGTCAACATGACAGGGTTGGATCTCAGTGC
CATCCAGGAAAGGCAGCTCTGGGTGGCTTTTCAGTGCTGCAGAGACTATGAAAATGTTCC
AGCAGCAGATCCCAGTGGAAGCCAGCAGCAGGCTGAGAAAGATGTGCCATCCTCAAACA
TAGGTCATGTCGAGGACAAGACAGATGATCCCGGGACCCATGTCCAATGTGTCAAAAGG
ACATTCCTTGCTTCAGGGGATTATGCAGACTTTCAGCCATTCACACAGAGTGAGGACAGT
CAGATGAAACATAGAGAAGAGATGTCAAATGAGGACTCCAGTGACTATGAAAATGTGCTA
ACTGCCAAGTTAGGAGGCAGGGACTCTGAGCAGGGGCCTGGCACTCAGCTCCTTCCTGA

TGAATGA (SEQ ID NO: 3)

By means of an example, human LAX1 protein sequence is annotated under NCBI Genbank accession numbers NP_060243.2, NP_00112966.2, NP_001269807.

As an example, amino acid sequence of NP_060243.2 is further reproduced below:

MDGVTPTLSTIRGRTLESSTLHVTPRSLDRNKDQITNIFSGFAGLLAILLVVAVFCILWNWNKR
KKRQVPYLRVTVMPLLTLPQTRQRAKNIYDILPWRQEDLGRHESRSMRIFSTESLLSRNSESP
EHVPSQAGNAFQEHTAHIHATEYAVGIYDNAMVPQMCGNLTPSAHCINVRASRDCASISSED
SHDYVNVPTAEEIAETLASTKSPSRNLFVLPSTQKLEFTEERDEGCGDAGDCTSLYSPGAED
SDSLSNGEGSSQISNDYVNMTGLDLSAIQERQLWVAFQCCRDYENVPAADPSGSQQQAEKD
VPSSNIGHVEDKTDDPGTHVQCVKRTFLASGDYADFQPFTQSEDSQMKHREEMSNEDSSDY
ENVLTAKLGGRDSEQGPGTQLLPDE (SEQ ID NO: 4)

By means of an example, human LAX1 gene is annotated under NCBI Genbank Gene ID 54900.

The reference to "IKZF3" ("IKAROS family zinc finger 3") denotes the IKZF3 markers, peptides, polypeptide, protein, or nucleic acid, as commonly known under said designation in the art. By means of additional guidance, IKZF3 is also known as "AIO", "AIOLOS", "IMD84", and "ZNFN1A3". The terms denote IKZF3 nucleic acids as well as IKZF3 peptides, polypeptides and proteins as apparent from the context.

By means of an example, human IKZF3 mRNA is annotated under NCBI Genbank (http://www.ncbi.nlm.nih.gov/) accession numbers
XM_047435625.1 (isoform X1), NM_001284516.1 (isoform 14), NM_001257409.2 (isoform 8), NM_001257408.2 (isoform 7), NM_001257413.2 (isoform 12), NM_001257412.2 (isoform 11), NM_001257411.2 (isoform 10), NM_001257410.2 (isoform 9), NM_001257414.2 (isoform 13), NM_183231.3 (isoform 5), NM_183228.3 (isoform 2), NM_001284515.2 (isoform 14), NM_183232.3 (isoform 6), NM_183229.3 (isoform 3), NM_183230.3 (isoform 4), NM_012481.5 (isoform 1), NM_001284514.2 (isoform 14)

Nucleotides 187 (start codon) to 1497 (stop codon) of NM_001257409.2 constitute the IKZF3 coding sequence (CDS). By means of an example, nucleotides 187 to 1497 of NM_001257409.2 are reproduced below:

ATGGAAGATATACAAACAAATGCGGAACTGAAAAGCACTCAGGAGCAGTCTGTGCCCGC
AGATGATTCAATGAAAGTGAAAGATGAATACAGTGAAAGAGATGAGAATGTTTTAAAGTCA
GAACCCATGGGAAATGCAGAAGAGCCTGAAATCCCTTACAGCTATTCAAGAGAATATAAT
GAATATGAAAACATTAAGTTGGAGAGACATGTTGTCTCATTCGATAGTAGCAGGCCAACC
AGTGGAAAGATGAACTGCGATGTGTGTGGATTATCCTGCATCAGCTTCAATGTCTTAATG
GTTCATAAGCGAAGCCATACTGGTGAACGCCCATTCCAGTGTAATCAGTGTGGGGCATCT
TTTACTCAGAAAGGTAACCTCCTCCGCCACATTAAACTGCACACAGGGGAAAAACCTTTT
AAGTGTCACCTCTGCAACTATGCATGCCAAAGAAGAGATGCGCTCACGGGGCATCTTAG

GACACATTCTGCAAGTGCGGAGGCAAGACACATCAAAGCAGAGATGGGAAGTGAAAGAG
CTCTCGTACTGGACAGATTAGCAAGCAATGTGGCAAAACGAAAAAGCTCAATGCCTCAGA
AATTCATTGGTGAGAAGCGCCACTGCTTTGATGTCAACTATAATTCAAGTTACATGTATGA
GAAAGAGAGTGAGCTCATACAGACCCGCATGATGGACCAAGCCATCAATAACGCCATCA
GCTATCTTGGCGCCGAAGCCCTGCGCCCCTTGGTCCAGACACCGCCTGCTCCCACCTCG
GAGATGGTTCCAGTTATCAGCAGCATGTATCCCATAGCCCTCACCCGGGCTGAGATGTC
AAACGGTGCCCCTCAAGAGCTGGAAAAGAAAAGCATCCACCTTCCAGAGAAGAGCGTGC
CTTCTGAGAGAGGCCTCTCTCCCAACAATAGTGGCCACGACTCCACGGACACTGACAGC
AACCATGAAGAACGCCAGAATCACATCTATCAGCAAAATCACATGGTCCTGTCTCGGGCC
CGCAATGGGATGCCACTTCTGAAGGAGGTTCCCCGCTCTTACGAACTCCTCAAGCCCCC
GCCCATCTGCCCAAGAGACTCCGTCAAAGTGATCAACAAGGAAGGGGAGGTGATGGATG
TGTATCGGTGTGACCACTGCCGCGTCCTCTTCCTGGACTATGTGATGTTCACGATTCACA
TGGGCTGCCACGGCTTCCGTGACCCTTTCGAGTGTAACATGTGTGGATATCGAAGCCAT
GATCGGTATGAGTTCTCGTCTCACATAGCCAGAGGAGAACACAGAGCCCTGCTGAAGTG
A (SEQ ID NO: 5)

By means of an example, human IKZF3 protein sequence is annotated under NCBI Genbank accession numbers XP_047291581.1, NP_001271445.1, NP_001244338.1, NP_001244337.1, NP_001244342.1, NP_001244340.1, NP_001244339.1, NP_001244343.1, NP_899054.1, NP_899051.1, NP_001271444.1, NP_899055.1, NP_899052.1, NP_899053.1, NP_036613.2, NP_001271443.1.

As an example, amino acid sequence of NP_001244338.1 is further reproduced below:

MEDIQTNAELKSTQEQSVPADDSMKVKDEYSERDENVLKSEPMGNAEEPEIPYSYSREYNEY
ENIKLERHVVSFDSSRPTSGKMNCDVCGLSCISFNVLMVHKRSHTGERPFQCNQCGASFTQ
KGNLLRHIKLHTGEKPFKCHLCNYACQRRDALTGHLRTHSASAEARHIKAEMGSERALVLDR
LASNVAKRKSSMPQKFIGEKRHCFDVNYNSSYMYEKESELIQTRMMDQAINNAISYLGAEALR
PLVQTPPAPTSEMVPVISSMYPIALTRAEMSNGAPQELEKKSIHLPEKSVPSERGLSPNNSGH
DSTDTDSNHEERQNHIYQQNHMVLSRARNGMPLLKEVPRSYELLKPPPICPRDSVKVINKEG
EVMDVYRCDHCRVLFLDYVMFTIHMGCHGFRDPFECNMCGYRSHDRYEFSSHIARGEHRAL
LK (SEQ ID NO: 6)

By means of an example, human IKZF3 gene is annotated under NCBI Genbank Gene ID 22806.

The reference to "ITGAL" ("integrin subunit alpha L") denotes the ITGAL markers, peptide, polypeptide, protein or nucleic acid as commonly known under designation in the art. By means of additional guidance, ITGAL is also known as "CD11A", "LFA-1", "LFA1A". The terms denote ITGAL nucleic acids, as well as ITGAL peptides, polypeptides and proteins, as apparent from the context. By means of an example, human ITGAL mRNA is annotated under NCBI Genbank (http://www.ncbi.nlm.nig.gov/) accession number NM_002209.3, XM_005255.13.1, XM_006721044.2, NM_001114380.2, XM_047434073.1, XM_047434072.1. By means of an example, human ITGAL protein is annotated under NCBI Genbank accession numbers NP_002200.2, XP_005255370.1, XP_006721107.1, NP_001107852.1, XP_047290029.1, XP_047290028.1.

The reference to "CD3G" ("CD3 gamma subunit of T-cell receptor complex") denotes the CD3G markers, peptide, polypeptide, protein or nucleic acid as commonly known under designation in the art. By means of additional guidance, CD3G is also known as "CD3-gamma", "IMD17" or "T3G". The terms denote CD3G nucleic acids, as well as CD3G peptides, polypeptides and proteins, as apparent from the context. By means of an example, human CD3G mRNA is annotated under NCBI Genbank (http://www.ncbi.nlm.nig.gov/) accession number NM_000073.3, XM_006718941.4, or XM_005271724.5. By means of an example, human CD3G protein is annotated under NCBI Genbank accession numbers NP_000064.1, NP_006719004.1, or XP_005271781.1. By means of example, human CD3G gene is annotated under NCBI Genbank ID: 917.

The reference to "CD3E" ("CD3 epsilon subunit of T-cell receptor complex") denotes the CD3E markers, peptide, polypeptide, protein or nucleic acid as commonly known under designation in the art. By means of additional guidance, CD3E is also known as ""CD3epsilon", "IMD18", "T3E" or "TCRE". The terms denote CD3E nucleic acids, as well as CD3E peptides, polypeptides and proteins, as apparent from the context. By means of an example, human CD3E mRNA is annotated under NCBI Genbank (http://www.ncbi.nlm.nig.gov/) accession number NM_000733.4. By means of an example, human CD3E protein is annotated under NCBI Genbank accession number NP_XXX NP_000724.1. By means of example, human CD3E gene is annotated under NCBI Genbank ID: 916.

The reference to "CXCR6" ("C-X-C motif chemokine receptor 6") denotes the CXCR6 markers, peptide, polypeptide, protein or nucleic acid as commonly known under designation in the art. By means of additional guidance, CXCR6 is also known as "BONZO", "CD186", "CDw186", "STRL33", "TYMSTR". The terms denote CXCR6 nucleic acids, as well as CXCR6 peptides, polypeptides and proteins, as apparent from the context. By means of an example, human CXCR6 mRNA is annotated under NCBI Genbank (http://www.ncbi.nlm.nig.gov/) accession number NM_001386436.1, NM_001386437.1, NM_006564.2, or NM_001386435.1. By means of an example, human CXCR6 protein is annotated under NCBI Genbank accession numbers NP_001373365.1, NP_00137336.1, NP_006555.1, or NP_001373364.1. By means of example, human CXCR6 gene is annotated under NCBI Genbank ID: 10663.

The reference to "SLAMF7" ("SLAM family member 7") denotes the SLAMF7 markers, peptide, polypeptide, protein or nucleic acid as commonly known under designation in the art. By means of additional guidance, SLAMF7 is also known as "19A", "CD319", "CRACC", "CS1". The terms denote SLAMF7 nucleic acids, as well as SLAMF7 peptides, polypeptides and proteins, as apparent from the context. By means of an example, human SLAMF7 mRNA is annotated under NCBI Genbank (http://www.ncbi.nlm.nig.gov/) accession number NM_001282588.2, NM_001282589.2, NM_001282590.2, NM_001282591.2, NM_001282592.2, NM_001282593.2, NM_001282594.2, NM_001282595.1, NM_001282596.2, or NM_021181.5. By means of an example, human SLAMF7 protein is annotated under NCBI Genbank accession numbers NP_001269517.1, NP_001269518.1, NP_001269519.1, NP_001269520.1, NP_001269521.1, NP_001269522.1, NP_001269523.1, NP_001269524.1, NP_001269525.1, or NP_067004.3. NP_XXX. By means of example, the human SLAMF7 gene is annotated under NCBI Genbank ID: 57823.

The reference to "MYO1G" ("myosin IG") denotes the MYOIG markers, peptide, polypeptide, protein or nucleic acid as commonly known under designation in the art. By means of additional guidance, MYOIG is also known as "HA", "HLA-HA2", or "MHAG". The terms denote MYO1G nucleic acids, as well as MYO1G peptides, polypeptides and proteins, as apparent from the context. By means of an example, human MYO1G mRNA is annotated under NCBI Genbank (http://www.ncbi.nlm.nig.gov/) accession number NM_033054.3. By means of an example, human MYO1G protein is annotated under NCBI Genbank accession numbers NP_149043.2. By means of example, the human MYO1G gene is annotated under NCBI Genbank ID: 64005.

The reference to "TIGIT" ("T cell immunoreceptor with Ig and ITIM domains") denotes the TIGIT markers, peptide, polypeptide, protein or nucleic acid as commonly known under designation in the art. By means of additional guidance, TIGIT is also known as "VSIG9", "VSTM3", or "WUCAM". The terms denote TIGIT nucleic acids, as well as TIGIT peptides, polypeptides and proteins, as apparent from the context. By means of an example, human TIGIT mRNA is annotated under NCBI Genbank (http://www.ncbi.nlm.nig.gov/) accession number NM_173799.4, XM_047447671.1, or XM_047447672.1. By means of an example, human TIGIT protein is annotated under NCBI Genbank accession numbers NP_776160.2, XP_047303627.1, or XP_047303628. By means of example, the TIGIT gene is annotated under NCBI Genbank ID: 201633.

The reference to "ICOS" ("inducible T cell costimulatory") denotes the ICOS markers, peptide, polypeptide, protein or nucleic acid as commonly known under designation in the art. By means of additional guidance, ICOS is also known as "AILIM", "CD278", or "CVID1". The terms denote ICOS nucleic acids, as well as ICOS peptides, polypeptides and proteins, as apparent from the context. By means of an example, human ICOS mRNA is annotated under NCBI Genbank (http://www.ncbi.nlm.nig.gov/) accession number NM_012092.4, or XM_047444022.1. By means of an example, human ICOS protein is annotated under NCBI Genbank accession numbers NP_036224.1, or XP_047299978.1. By means of example, the ICOS gene is annotated under NCBI Genbank ID: 29851.

The reference to "CLEC4C" ("C-type lectin domain family 4 member C") denotes the CLEC4C markers, peptide, polypeptide, protein or nucleic acid as commonly known under designation in the art. By means of additional guidance, CLEC4C is also known as "BDCA-2", "BDCA2", "CD303", "CLECSF11", "CLECSF7", "DLEC", "HECL", or "PRO34150". The terms denote CLEC4C nucleic acids, as well as CLEC4 peptides, polypeptides and proteins, as apparent from the context. By means of an example, human CLEC4C mRNA is annotated under NCBI Genbank (http://www.ncbi.nlm.nig.gov/) accession number NM_001371390.1, NM_001371391.1, NM_130441.3, or NM_203503.2. By means of an example, human CLEC4C protein is annotated under NCBI Genbank accession numbers NP_001358319.1, NP_001358320.1, NP_569708.1, or NP_987099.1. By means of example, the human CLEC4C gene is annotated under NCBI Genbank ID: 170482.

The reference to "CXCR3" ("C-X-C motif chemokine receptor 3") denotes the CXCR3 markers, peptide, polypeptide, protein or nucleic acid as commonly known under designation in the art. By means of additional guidance, CXCR3 is also known as "CD182", "CD183", "CKR-L2", "CMKAR3", "GPR9", "IP10-R", "Mig-R", or "MigR". The terms denote CXCR3 nucleic acids, as well as CXCR3 peptides, polypeptides and proteins, as apparent from the context. By means of an example, human CXCR3 mRNA is annotated under NCBI Genbank (http://www.ncbi.nlm.nig.gov/) accession number NM_001504.2, XM_047442010.1, NM_001142797.2, XM_005262257.4, XM_005262256.4, or XM_017029435.2. By means of an example, human CXCR3 protein is annotated under NCBI Genbank accession numbers NP_001495.1, XP_047297966.1, NP_001136269.1, XP_005262314.1, XP_00526313.1, XP_016884924.1. By means of example, the human CXCR3 gene is annotated under NCBI Genbank ID: 2833.

The reference to "TLR8" ("toll like receptor 8) denotes the TLR8 markers, peptide, polypeptide, protein or nucleic acid as commonly known under designation in the art. By means of additional guidance, TLR8 is also known as "CD288", "IMD98", "hTLR8". The terms denote TLR8 nucleic acids, as well as TLR8 peptides, polypeptides and proteins, as apparent from the context. By means of an example, human TLR8 mRNA is annotated under NCBI Genbank (http://www.ncbi.nlm.nig.gov/) accession number NM_016610.4, or NM_138636.5. By means of an example, human TLR8 protein is annotated under NCBI Genbank accession numbers NP_057694.2 or NP_619542.1. By means of example, the human TLR8 gene is annotated under NCBI Genbank ID: 51311.

The reference to "FCGR2A" ("Fc gamma receptor IIa") denotes the FCGR2A markers, peptide, polypeptide, protein or nucleic acid as commonly known under designation in the art. By means of additional guidance, FCGR2A is also known as "CD32", "CD32A", "CDw32", "FCG2", "FCGR2", FCGR2A1", "FcGR", "FcgammaRlla", or "IGFR2". The terms denote FCGR2A nucleic acids, as well as FCGR2A peptides, polypeptides and proteins, as apparent from the context. By means of an example, human FCFR2A mRNA is annotated under NCBI Genbank (http://www.ncbi.nlm.nig.gov/) accession number NM_001136219.3, NM_001375296.1, NM_001375297.1, or NM_021642.5. By means of an example, human FCGR2A protein is annotated under NCBI Genbank accession numbers NP_001129691.1, NP_001362225.1, NP_001362226.1, or NP_067674.2. By means of example, the human FCGR2A gene is annotated under NCBI Genbank ID: 2212.

The reference to "IDO1" ("indoleamine 2,3-dioxygenase 1") denotes the IDO1 markers, peptide, polypeptide, protein or nucleic acid as commonly known under designation in the art. By means of additional guidance, IDO1 is also known as "IDO", "IDO-1", or "INDO". The terms denote IDO nucleic acids, as well as IDO peptides, polypeptides and proteins, as apparent from the context. By means of an example, human IDO mRNA is annotated under NCBI Genbank (http://www.ncbi.nlm.nig.gov/) accession number NM_002164.6. By means of an example, human IDO protein is annotated under NCBI Genbank accession numbers NP_002155.1. By means of an example, the human IDO1 gene is annotated under NCBI Genbank ID: 3620.

The reference to "TRAF3IP3" ("TRAF3 interacting protein 3") denotes the TRAF3IP3 markers, peptide, polypeptide, protein or nucleic acid as commonly known under designation in the art. By means of additional guidance, TRAF31P3 is also known as "T3JAM". The terms denote TRAF3IP3 nucleic acids, as well as TRAF3IP3 peptides, polypeptides and proteins, as apparent from the context. By means of an example, human TRAF3IP3 mRNA is annotated under NCBI Genbank (http://www.ncbi.nlm.nig.gov/) accession number NM_001287754.2, NM_001320143.2, NM_001320144.2, NM_025228.4. By means of an example, human TRAF3IP3 protein is annotated under NCBI Genbank accession numbers NP_001274683.1, NP_001307072.1, NP_001307073.1, or NP_079504.2. By means of example, the human TRAF3IP3 gene is annotated under NCBI Genbank ID: 80342.

The reference to "PIK3CD" ("phosphatidylinositol-4,5-bisphosphate 3-kinase catalytic subunit delta") denotes the PIK3CD markers, peptide, polypeptide, protein or nucleic acid as commonly known under designation in the art. By means of additional guidance, PIK3CD is also known as "APDS", "IMD14", "IMD14A", "IMD14B", "P110DELTA", "PI3K", "ROCHIS", or "p110D". The terms denote PIK3CD nucleic acids, as well as PIK3CD peptides, polypeptides and proteins, as apparent from the context. By means of an example, human PIK3CD mRNA is annotated under NCBI Genbank (http://www.ncbi.nlm.nig.gov/) accession number NM_001350234.2, NM_001350235.1, or NM_005026.5. By means of an example, human PIK3CD protein is annotated under NCBI Genbank accession numbers NP_001337163.1, NP_001337164.1, NP_005017.3. By means of example, the human PIK3CD gene is annotated under NCBI Genbank ID: 5293.

The reference to "IGLV1.36" ("immunoglobulin lambda variable 1-36) denotes the IGLV1.36 markers, peptide, polypeptide, protein or nucleic acid as commonly known under designation in the art. By means of additional guidance, ITGAL is also known as "IGLV136", "V1-11". The terms denote IGLV1.36 nucleic acids, as well as IGLV1.36 peptides, polypeptides and proteins, as apparent from the context. By means of examples, the human IGLV1.36 gene is annotated under NCBI Genbank ID: 28826.

The reference to "TRAV14DV4" ("T cell receptor alpha variable 14/delta variable 4") denotes the TRAV14DV4 markers, peptide, polypeptide, protein or nucleic acid as commonly known under designation in the art. By means of additional guidance, TRAV14DV4 is also known as "hADV14S1", "TRAV14/DV4", "TCRAV6S1-hDV104S1". The terms denote TRAV14DV4 nucleic acids, as well as TRAV14DV4 peptides, polypeptides and proteins, as apparent from the context. By means of example, the human TRAV14DV4 gene is annotated under NCBI Genbank ID: 28669.

The reference to "TRAJ16" (T cell receptor alpha joining 16") denotes the ITGAL markers, peptide, polypeptide, protein or nucleic acid as commonly known under designation in the art. By means of additional guidance, The terms denote TRAJ16 nucleic acids, as well as TRAJ16 peptides, polypeptides and proteins, as apparent from the context. By means of an example, the human TRAJ16 gene is annotated under NCBI Genbank ID: 28739.

The reference to "IGHV1.3" ("immunoglobulin heavy variable 1-3") denotes the IGHV1.3 markers, peptide, polypeptide, protein or nucleic acid as commonly known under designation in the art. By means of additional guidance, IGHV1.3 is also known as "IGHV1-3", "IGHV13" or "VI-3B". The terms denote IGHV1.3 nucleic acids, as well as IGHV1.3 peptides, polypeptides and proteins, as apparent from the context. By means of example, the human IGHV1.3 gene is annotated under NCBI Genbank ID: 28473.

The reference to "IGHV4.28" ("immunoglobulin heavy variable 4-28") denotes the IGHV4.28 markers, peptide, polypeptide, protein or nucleic acid as commonly known under designation in the art. By means of additional guidance, IGHV4.28 is also known as "IGHV4-28", "IGHV428" or "VH". The terms denote IGHV4.28 nucleic acids, as well as IGHV4.28 peptides, polypeptides and proteins, as apparent from the context. By means of an example, the human IGHV4.28 gene is annotated under NCBI Genbank ID: 28400.

The reference to "IGHV4.31" ("immunoglobulin heavy variable 4-31") denotes the IGHV4.31 markers, peptide, polypeptide, protein or nucleic acid as commonly known under designation in the art. By means of additional guidance, IGHV4.31 is also known as "IGHV4-31", or "IGHV431". The terms denote IGHV4.31 nucleic acids, as well as IGHV4.31 peptides, polypeptides and proteins, as apparent from the context. By means of an example, the human IGHV4.31 gene is annotated under NCBI Genbank ID: 28396.

The reference to "IGHV4.4" ("immunoglobulin heavy variable 4-4") denotes the IGHV4.4 markers, peptide, polypeptide, protein or nucleic acid as commonly known under designation in the art. By means of additional guidance, IGHV4.4 is also known as "IGHV4-4", or "IGHV44". The terms denote IGHV4.4 nucleic acids, as well as IGHV4.4 peptides, polypeptides and proteins, as apparent from the context. By means of an example, the human IGHV4.4 gene is annotated under NCBI Genbank ID: 28401.

A skilled person can appreciate that any sequences represented in sequence databases or in the present specification may be of precursors of markers, peptides, polypeptides, proteins or nucleic acids and may include parts which are processed away from mature molecules.

Unless otherwise apparent from the context, reference herein to any marker, gene, peptide, polypeptide or protein, or fragment thereof may generally also encompass modified forms of said marker, gene, peptide, polypeptide, or protein, or fragment thereof, such as genetic alterations, such as mutations, or bearing post-expression modifications including, for example, phosphorylation, glycosylation, lipidation, methylation, cysteinylation, sulphonation, glutathionylation, acetylation, oxidation, and the like.

The reference herein to any marker, gene, peptide, polypeptide or protein also encompasses fragments thereof. Hence, the reference herein to measuring (or measuring the quantity of), determining the presence, or determining the expression level of any one marker, gene, peptide, polypeptide or protein may encompass measuring, determining the presence or determining the expression level of the marker, gene, peptide, polypeptide, or protein, such as, e.g. measuring any mature and/or processed soluble/secreted form(s) thereof (e.g., plasma circulating form(s)) and/or measuring one or more fragments thereof.

For example, any marker, gene, peptide, polypeptide or protein, and/or one or more fragments thereof may be measured collectively, such that the measured quantity corresponds to the sum amounts of the collectively measured species. In a further example, any marker, gene, peptide, polypeptide or protein and/or one or more fragments thereof may be measured each individually.

The term "fragment" with reference to a peptide, polypeptide, or protein generally denotes a N- and/or C-terminally truncated form of the peptide, polypeptide, or protein. Preferably, a fragment may comprise at least about 30%, e.g., at least about 50% or at least about 70%, preferably at least about 80%, e.g., at least about 85%, more preferably at least about 90%, and yet more preferably at least about 95% or even about 99% of the amino acid sequence length of said peptide, polypeptide, or 10 protein. For example, insofar not exceeding the length of the full-length peptide, polypeptide, or protein, a fragment may include a sequence of ≥ 5 consecutive amino acids, or ≥ 10 consecutive amino acids, or ≥ 20 consecutive amino acids, or ≥ 30 consecutive amino acids, e.g., ≥ 40 consecutive amino acids, such as for example ≥ 50 consecutive amino acids, e.g., ≥ 60, ≥ 70, ≥ 80, ≥ 90, ≥ 100, ≥ 200, ≥ 300 or ≥ 400 consecutive amino acids of the corresponding full-length peptide, polypeptide, or protein.

The reference herein to any protein, polypeptide or peptide may also encompass variants thereof. The term "variant" of a protein, polypeptide or peptide refers to proteins, polypeptides or peptides the sequence (i.e. amino acid sequence) of which is substantially identical (i.e. largely but not wholly identical) to the sequence of said recited protein or polypeptide, e.g. at least about 80% identical or at least about 85% identical, e.g. preferably at least about 90% identical, e.g., at least 91% identical, 92% identical, more preferably at least about 93% identical, e.g. at least 94% identical, even more preferably at least about 95% identical, e.g., at least 96% identical, yet more preferably at least about 97% identical, e.g. at least 98% identical, and most preferably at least 99% identical. Preferably, a variant may display such degrees of identity to a recited protein, polypeptide or peptide when the whole sequence of the recited protein, polypeptide or peptide is querried in the sequence alignment (i.e, overall sequence identity).

A variant of a protein, polypeptide or peptide may be a homologue (e.g, orthologue or paralogue) of said protein, polypeptide or peptide. As used herein, the term "homology" generally denotes structural similarities between macromolecules, particularly between two proteins or polypeptides, from same or different taxons, wherein said similarity is due to shared ancestry.

Where the present specification refers to or encompasses fragments and/or variants of proteins, polypeptides or peptides, this preferably denotes variants and/or fragments which are "functional", i.e., which at least partly retain the biological activity or intended functionality of the respective proteins, polypeptides or peptides. Preferably, a functional fragment and/or variant may retain at least about 20%, e.g., at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the intended biological activity or functionality compared to the corresponding protein, polypeptide or peptide.

In some embodiments, the risk score obtained in any of the methods of the application can further be improved by immunohistochemistry or protein expression data of the tumor of the subject. For example, the Cytokine 30-Plex Human Panel and immunohistochemistry stainings can be used for additional qualitative and/or quantitative assessment of the sample of the subject.

Also provided is a computer-implemented method for predicting and/or determining whether a subject diagnosed with cancer will respond to treatment with an immunotherapeutic agent that is an immune checkpoint inhibitor or a combination of immune checkpoint inhibitors, wherein a sample obtained from said subject is analyzed wherein the sample contains or is suspected to contain tumor cells, the method comprising: (a) providing the quantified expression level of the biomarkers CD247, LAX1 and IKZF3, said biomarkers being quantified in the sample of the subject; (b) optionally, providing the quantified expression level of the biomarkers CD3G and ITGAL and/or one or more of the biomarkers selected from the group consisting of CD3E, TIGIT, PIK3CD, IGHV1.3, IGHV4.4 in the sample of the subject, (c) normalizing the quantified expression levels whereby normalization occurs via comparison with data obtained from corresponding assessments and expression levels from a reference set, (d) classifying, whether the normalized values of step (c) exceed a predetermined threshold, (e) obtaining a risk score of said normalized values, wherein said score is calculated using a predictive algorithm or machine learning model, and wherein the risk score represents the likelihood for the response to the immunotherapeutic agent by the subject.

Disclosed herein is also a kit for predicting and/or determining the response of a subject to an immunotherapeutic agent is disclosed. In some embodiments, said kit comprises means for measuring the expression level of at least 3, preferably at least 4 or 5, even more preferably at least 10 or all of the biomarkers selected from the list consisting of CD247, LAX1, IKZF3, ITGAL, CD3G, CD3E, CXCR6, SLAMF7, MYO1G, TIGIT, ICOS, CLEC4C, CXCR3, TLR8, FCGR2A, IDO1, TRAF3IP3, PIK3CD, IGLV1.36, TRAV14DV4, TRAJ16, IGHV1.3, IGHV4.28, IGHV4.31, IGHV4.4, in a sample of the subject. Optionally, said kit further comprises a reference value or threshold value for one or more of the biomarkers selected from the list consisting of CD247, LAX1, IKZF3, ITGAL, CD3G, CD3E, CXCR6, SLAMF7, MYO1G, TIGIT, ICOS, CLEC4C, CXCR3, TLR8, FCGR2A, IDO1, TRAF3IP3, PIK3CD, IGLV1.36, TRAV14DV4, TRAJ16, IGHV1.3, IGHV4.28, IGHV4.31, IGHV4.4.

In an aspect of the invention, the use of a kit for predicting and/or determining the response of a subject to an immunotherapeutic agent is provided, wherein said kit comprises means for measuring the expression level of the biomarkers CD247, LAX1 and IKZF3 in a sample of the subject wherein the sample contains or is suspected to contain tumor cells; optionally, means for measuring the expression level of the biomarkers CD3G and ITGAL and/or one or more of the biomarkers selected from the list consisting of CD3E, TIGIT, PIK3CD, IGHV1.3, IGHV4.4 in a sample of the subject wherein the sample contains or is suspected to contain tumor cells. Optionally, said kit further comprises a reference value or threshold value for the biomarkers CD247, LAX1 and IKZF3. Said kit may further also comprise a reference value or threshold value for the biomarkers CD3G and ITGAL, and/or for one or more of the biomarkers selected from the list consisting of CD3E, TIGIT, PIK3CD, IGHV1.3, IGHV4.4.

In still some further embodiments, the kits of the present application may comprise ready-to-use substrate solutions, wash solutions, dilution buffers and instructions. The kit may also comprise positive and/or negative control samples. In some embodiments, the kit comprises instructions for use. Preferably, the instructions for use included in the kit are unambiguous, concise and comprehensible to those skilled in the art. The instructions typically provide information on kit contents, how to collect the sample, methodology, experimental read-outs and interpretation thereof and cautions and warnings.

The terms "kit of parts" and "kit" as used throughout this specification refer to a product containing components necessary for carrying out the specified methods (e.g., method for predicting and/or determining the response of a subject to an immunotherapeutic agent, method for deciding whether a subject is eligible for an immunotherapeutic treatment or method for determining whether a subject is predisposed for responding to an immunotherapeutic treatment), packed so as to allow their transport and storage. Materials suitable for packing the components comprised in a kit include crystal, plastic (e.g., polyethylene, polypropylene, polycarbonate), bottles, flasks, vials, ampules, paper, envelopes, or other types of containers, carriers or supports. Where a kit comprises a plurality of components, at least a subset of the components (e.g., two or more of the plurality of components) or all of the components may be physically separated, e.g. comprises in or on separate containers, carriers or supports. The components comprised in a kit may be sufficient or may not be sufficient for carrying out the specified methods, such that external reagents or substances may not be necessary or may be necessary for performing the methods, respectively. Typically, kits are employed in conjunction with standard laboratory equipment, such as liquid handling equipment, environment (e.g., temperature) controlling equipment, analytical instruments, etc. The kits may also include some or all of solvents, buffers (such as for example without limitation histidine-buffers, citrate-buffers, succinate-buffers, acetate-buffers, phosphate-buffers, formate buffers, benzoate buffers, TRIS ((Tris(hydroxymethyl)-aminomethan) buffers or maleate buffers, or mixtures thereof), enzymes (such as for example but without limitation thermostable DNA polymerase), detectable labels, detection reagents, and control formulations (positive and/or negative), useful in the specified methods.

Typically, the kits may also include instructions for use thereof, such as a printed insert or on a computer readable medium. The terms may be used interchangeably with the term "article of manufacture", which broadly encompasses any man-made tangible structural product, when used in the present context.

In particular embodiments, the kit further comprises a computer readable storage medium having recorded thereon one or more programs for carrying out the methods as taught herein. Further, also the use of said kits is provided, in particular for predicting and/or determining the response of a subject to an immunotherapeutic agent, or for deciding whether a subject is eligible for an immunotherapeutic treatment or for determining whether a subject is predisposed for responding to an immunotherapeutic treatment.

Further provided is the use of a kit comprising means for measuring the expression level of the biomarkers CD247, LAX1 and IKZF3 in a sample of the subject wherein the sample contains or is suspected to contain tumor cells in a method for determining and/or predicting the response or resistance to treatment with an immunotherapeutic agent that is an immune checkpoint inhibitor or a combination of immune checkpoint inhibitors in a subject diagnosed with cancer as taught herein. In a further embodiment, said use of a kit is provided wherein the kit further comprises means for measuring the expression level of the biomarkers CD3G and ITGAL in a sample of the subject wherein the sample contains or is suspected to contain tumor cells. In another embodiment, said use of a kit is provided wherein the kit further comprises means for measuring the expression level of the biomarkers CD3E, TIGIT, PIK3CD, IGHV1.3, IGHV4.4 in a sample of the subject wherein the sample contains or is suspected to contain tumor cells. In some embodiments, said use of a kit is provided wherein the kit further comprises a reference value or threshold value for the biomarkers CD247, LAX1 and IKZF3, and/or a reference value or threshold value for the biomarkers CD3G and ITGAL, and/or a reference value or threshold value for the biomarkers CD3E, TIGIT, PIK3CD, IGHV1.3 and IGHV4.4.

The inventors of the present application identified several biomarkers for predicting and/or determining the response of a subject to an immunotherapeutic agent or for deciding whether a subject is eligible for treatment with an immunotherapeutic agent. More specifically, the biomarkers were identified to be specifically suitable for predicting responses after treatment with immune checkpoint inhibitors. The immunotherapeutic agent is therefore an immune checkpoint inhibitor or a combination of several immune checkpoint inhibitors. Even further, the immune checkpoint inhibitor is selected from the group consisting of a PD-1 targeting agent, a PD-L1 targeting agent, a CTLA-4 targeting agent, and a combination thereof. In some further preferred embodiments, the immunotherapeutic treatment is a PD-1 targeting agent or a PD-L1 targeting agent in combination with a CTLA-4 targeting agent. In some embodiments, the PD-1 targeting agents are monoclonal antibodies against PD-1, such as for example pembrolizumab, nivolumab or cemiplimab. In some embodiments, the PD-L1 targeting agents are monoclonal antibodies against PD-L1, such as for example Atezolizumab, Avelumab, Durvalumab, Nivolumab, or Pembrolizumab. In some embodiments, the CTLA-4 targeting agents are monoclonal antibodies against CTLA-1, such as for example Ipilimumab. In still some further embodiments, a PDL-1 targeting agent, such as Atezolizumab, Avelumab, Durvalumab, Nivolumab, or Pembrolizumab, is combined with a CTLA-4 targeting agent, such as Ipilimumab. Immunotherapeutic drugs, like immune checkpoint inhibitors, target certain immune cells that need to be activated or inactivated to start an immune response. Monoclonal antibodies that target either PD-1 or PD-L1 can block these proteins, which are present on respectively T-cells and normal (or cancer) cells, and boost the immune response against cancer cells. These drugs are helpful in treating different types of cancer, including bladder cancer, non-small cell lung cancer, and Merkel cell skin cancer. CTLA-4 is another protein on some T cells that, just like PD-1, acts as a type of switch to keep the immune system in check. Anti-CTLA4 treatment is often used to treat melanoma of the skin and some other cancers.

The PD-1 targeting agents, PD-L1 targeting agents or CTLA-4 targeting agents can be any targeting agent known in the art, such as an antibody or a fragment thereof that recognizes and blocks PD-1, PD-L1 or CTLA-4. In some embodiments, the PD-1 targeting agents are monoclonal antibodies against PD-1, such as for example pembrolizumab, nivolumab or cemiplimab. In some embodiments, the PD-L1 targeting agents are monoclonal antibodies against PD-L1, such as for example Atezolizumab, Avelumab, Durvalumab, Nivolumab, or Pembrolizumab. In some embodiments, the CTLA-4 targeting agents are monoclonal antibodies against CTLA-1, such as for example Ipilimumab. In still some further embodiments, a PDL-1 targeting agent, such as Atezolizumab, Avelumab, Durvalumab, Nivolumab, or Pembrolizumab, is combined with a CTLA-4 targeting agent, such as Ipilimumab.

Depending on the type of cancer another ICI treatment is advised. A combination therapy or a monotherapy is possible. The set of predictive biomarkers of the current invention are preferably used to determine whether a subject that is diagnosed with cancer will respond to treatment with an immunotherapeutic agent, in particular an immune checkpoint inhibitor or a combination of checkpoint inhibitors. More preferably the set of predictive biomarkers of current invention is used to determine the response of a subject when receiving an immunotherapeutic treatment, that is treatment with an immune checkpoint inhibitor. Most preferably the set of predictive biomarkers of current invention is used to determine the response of a subject when receiving an immune checkpoint inhibitor that is selected from the group consisting of a PD-1 targeting agent, a PD-L1 targeting agent, a CTLA-4 targeting agent, and a combination thereof..

The sample disclosed herein is a sample that contains or is suspected to contain tumor cells. In some embodiments, the sample contains tumor cells. In some embodiments, the sample is a tumor tissue sample or a fluid sample comprising tumor cells. In some embodiments, the sample is a tumor tissue sample derived from subjects diagnosed with cancer. In some embodiments, the sample is a fluid sample, such as a blood sample, comprising tumor cells, in particular circulating tumor cells. In some embodiments, the sample is a sample comprising circulating tumor cells. The expression level of the biomarkers is thus assessed in a biological sample that contains or is suspected to contain cancer cells. The tissue sample may be, for example, a tissue resection, a tissue biopsy, or metastatic lesion obtained from a patient suffering from, suspected to suffer from, or diagnosed with cancer. Preferably, the sample is a sample of tissue, a resection or biopsy of a tumor, known or suspected metastatic cancer lesion or section. Methods of obtaining biological samples including tissue resections, biopsies, and body fluids, e.g., blood samples comprising cancer/tumor cells, are well known in the art.

In some embodiments, the sample is a tumor tissue sample, such as a fresh-frozen tumor tissue sample, a fresh tumor tissue sample, or a formalin-fixed paraffin-embedded (FFPE) tumor tissue sample. Isolation of nucleic acids, such as RNA or DNA, from tumor tissue samples can be performed using any technique known in the art. For example, RNA isolation from a tumor tissue sample, such as an FFPE tumor tissue sample, can be performed using the QIAGEN RNeasy FFPE kit (standard kit) following Manufacturer's instructions (Qiagen).

In some embodiments, the sample is a fluid sample comprising tumor cells, in particular circulating tumor cells. In some embodiments, the fluid sample is a blood sample, e.g. a peripheral blood sample, known or suspected to comprise circulating cancer cells. The sample may comprise both cancer cells, i.e. tumor cells, and non-cancerous cells, and, in certain embodiments, comprises both cancerous and non-cancerous cells. In some embodiments, the sample is a sample of isolated circulating tumor cells. Isolation of circulating tumor cells from liquid samples, such as a blood sample, can be performed using any technique known in the art. For example, isolation of circulating tumor can be performed using the EasySep^{™} Direct Human CTC enrichment kit (Stemcell Technologies), or the CTC ready-to-use CTC AdnaTest of Qiagen (Qiagen). RNA and/or DNA isolation from the isolated CTCs can for example be performed using the AllPrep RNA/mRNA Nano kit (Qiagan), or the RNAeasy Tissue/Cells Advances mini kit (Qiagen).

In some embodiments, the sample obtained from the patient is collected prior to beginning any immunotherapy or other treatment regimen or therapy, e.g. chemotherapy, or radiation therapy for the treatment of cancer or the management or amelioration of a symptom thereof. Therefore, in some embodiments, the sample is collected before the administration of immunotherapeutic agents or other agents, or the start of immunotherapy or other treatment regimen.

In some embodiments the subject is a subject diagnosed with cancer. The cancer can be selected from bladder cancer, kidney cancer, liver cancer, lung cancer, pancreatic cancer, prostate cancer, thyroid cancer, uterine cancer, ovarian cancer, colorectal cancer, breast cancer, head and neck cancer, skin cancer; even more preferably cancer patients diagnosed with skin cancer or melanoma, lung cancer such as lung adenocarcinoma, lung squamous cell carcinoma, non-small cell lung carcinoma (NSCLC). In a preferred embodiment, the subject is a subject diagnosed with melanoma and/or lung cancer. In a further preferred embodiment, the subject is diagnosed with melanoma. In some embodiments, the subject is diagnosed with stage II, stage III or stage IV melanoma.

The current invention is preferably depicted for patients diagnosed with melanoma. These patients may have already undergone an anti-PD-1, anti-PD-L1 and/or anti-CTLA-4 treatment, are undergoing such a treatment or are planned to be treated with such drugs. The set of predictive biomarkers is optimized for such patients, and predicts with high specificity and sensitivity the response of the patient's tumor towards such a treatment or the chance of developing progressive disease or early death.

In a preferred embodiment, the different set of biomarkers of the current invention are used for the development of novel immunotherapeutic drugs.

Some embodiments of the application relate to the use of the previously described methods for predicting responsiveness or non-responsiveness of a subject undergoing one or more immunotherapeutic treatments. Preferably, for predicting responsiveness or non-responsiveness of a subject undergoing anti-PD-1/PD-L1 monotherapy, or in combination with anti-CTLA-4 therapy.

Prognostically identifying which patients are going to experience progression or be non-responders to such immunotherapeutic treatments is of medical and economical interest. An optimal patient specific therapy can be determined which assures a better and safer therapy.

In some embodiments, a method for determining the response of a tumor to an immunotherapeutic treatment is disclosed wherien the method comprises the steps of quantitative assessment of the expression level of a plurality of biomarkers, preferably protein coding genes involved in responsiveness or resistance to the immunotherapeutic treatment; and based on this quantitative assessment classifying the tumor as a responder, a partial responder or a non-responder to the therapy.

In some embodiments, the method further comprises the step of determining immunohistochemistry data for the tumor.

Preferably immunohistochemistry data is included in the characterization of the tumor and tumor microenvironment. This extra dataset makes the identification of the tumor is a responder, partial responder or non-responder more reliable and valid, as repeated measurements generate a similar output.

In a more preferred embodiment, the method further comprises the step of determining one or more possible immunotherapeutic treatment therapies.

Next to the targeted ICI treatment other immunotherapeutic treatment therapies could be addressed in the treatment of cancer patients leading to a positive outcome of the cancer patients treatment.

### EXAMPLES

The invention is further described by the following examples which further illustrate the invention

### Methods

### Study design, patient cohorts, and response assessment.

A total of 273 metastatic melanoma patients treated with PD1/PD-L1 inhibitor or its combinations with CTLA4 inhibitor were selected for analysis. These included 210 patients from 4 interventional clinical trials deposited in the GEO and dbGaP databases (studies 356761 [doi: 10.1016/j.cell.2017.09.028], 522397 [doi: 10.1016/j.ccell.2019.01.003], phs000452_01 [doi: 10.1038/s41591-019-0654-5] and GSE78220 [doi: 10.1016/j.cell.2016.02.065]) and 63 patients from an on-going observational retrospective study in 3 hospitals in Belgium and Austria (NCT04860076). The patients enrolled into the study were treated with Nivolumab or Pembrolizumab, or their combinations with Ipilimumab. We complied with all relevant ethical regulations for work with human participants, and informed consent was obtained from all patients. The protocol was approved by the independent ethics committees at each participating center. Response was assessed by RECIST v1.1 (PD - progressive disease, SD - stable disease, PR - partial response, CR - complete response). Patients who did not reach an evaluable RECIST score as defined by CR/PR/SD/PD were excluded from the study. Patients which had PFS < 90 days and OS < 180 were categorized as early death (ED) subgroup. To ensure translatability of the results, dbGaP datasets were used as the sole discovery cohort and data from the NCT04860076 clinical study served solely as validation cohort.

### RNA extraction and whole transcriptome sequencing.

For the NCT04860076 the total RNA was extracted from FFPE blocks using QIAGEN RNeasy FFPE kit (standard kit) following Manufacturer's instructions (Qiagen, Hilden, Germany). All FFPE blocks were obtained from the biopsies extracted prior to the start of treatment with immune checkpoint inhibitors. On average 5-7 FFPE slices were used for RNA extraction. RNA samples were quantified using Qubit 2.0 Fluorometer RNA assay (Invitrogen, Carlsbad, CA, USA) and RNA integrity was checked with Agilent 4200 TapeStation (Agilent Technologies, Palo Alto, CA, USA). Samples with a DV200 score less than 30% were excluded from further analysis. RNA samples of satisfactory quality were treated with TURBO DNase (Thermo Fisher Scientific, Waltham, MA, USA) to remove DNA following manufacturer's protocol. rRNA depletion was performed using QIAGEN FastSelect rRNA HMR Kit (Qiagen, Hilden, Germany). RNA sequencing library preparation uses NEBNext Ultra II RNA Library Preparation Kit for Illumina by following the manufacturer's recommendations (NEB, Ipswich, MA, USA). Briefly, enriched RNAs are fragmented for 15 minutes at 94 °C. First strand and second strand cDNA are subsequently synthesized. cDNA fragments are end repaired and adenylated at 3'ends, and universal adapters are ligated to cDNA fragments, followed by index addition and library enrichment with limited cycle PCR. Sequencing libraries were validated using the Agilent Tapestation 4200 (Agilent Technologies, Palo Alto, CA, USA), and quantified using Qubit 2.0 Fluorometer (Invitrogen, Carlsbad, CA) as well as by quantitative PCR (Applied Biosystems, Carlsbad, CA, USA).

The sequencing libraries were multiplexed and clustered on a lane of a flowcell and loaded on the Illumina NovaSeq S4 instrument according to manufacturer's instructions. The samples were sequenced for a target of ~50 million reads using a 2x150 Paired End (PE) configuration. Image analysis and base calling were conducted by the HiSeq Control Software (HCS). Raw sequence data (.bcl files) generated from Illumina HiSeq was converted into FASTQ files and de-multiplexed using Illumina's bcl2fastq 2.17 software. One mis-match was allowed for index sequence identification.

Similar procedures were used for RNA extraction and sequencing in the studies 356761, 522397, phs000452_01 and GSE78220 from the GEO and dbGaP databases [doi: 10.1016/j.cell.2017.09.028, 10.1016/j.ccell.2019.01.003], 10.1038/s41591-019-0654-5, 10.1016/j.cell.2016.02.065].

### RNA-seq data processing and normalization.

Whole-transcriptome profiles were generated for 63 patients from the NCT04860076 study and combined with the whole transcriptome profiles from the dbGaP dataset. Obtained FASTQ files were processed using a custom internal pipeline. Briefly, raw bulk RNA-seq reads were processed using nf-core/rnaseq pipeline to transform the raw RNA reads and to obtain the gene expression matrices. The workflow included a series of steps of read quality control and read trimming (FastQC, Trim Galore!), aligning reads against the human reference genome GRCh38 (STAR), calculating counts relative to genes (featureCounts), and quality control on the results (RSeQC, Qualimap, Preseq, edgeR, MultiQC). The pipeline was adjusted and fine-tuned to best suit the needs of downstream analyses and available resources. ComBat-seq [doi: 10.1093/nargab/Iqaa078] was used to normalize the data across all datasets.

### Principal component analysis (PCA) and principal variance component analysis (PVCA).

PCA was conducted on the 24,3769 genes detected within the dataset using the prcomp() function. PCA analysis was also conducted on MM cohorts from dbGaP and the NCT04860076 study. PVCA is a hybrid method that combined PCA and VCA to quantify the contribution of known variables to the global variance observed in the transcriptional data. PVCA analysis was conducted using the pvca package (v1.18.0. Bioconductor). The PCA was used to verify comparability of post-combat normalized data and identify potential confounding variables that may impact the analyses.

### Predictive signature discovery and validation using machine learning.

An ensemble model with LASSO-based learner was used on the discovery cohort (dbGaP) to identify predictive markers of response to treatment. Ensemble models are known to be more robust and less affected by initial seeds. This regression analysis method concurrently performs variable selection and regularization to improve accuracy. To build the base learners, 1000 LASSO models were built using different seeds. In each model, hyper-parameter selection was conducted by fivefold cross-validation. The final ensemble model was built by taking the average of the coefficient of the 1000 LASSO models. In more detail, in each LASSO model, each gene is assigned a coefficient (if a gene is not selected by this model, the coefficient will be 0). A gene's coefficient in the final ensemble model is then obtained by taking the average of its coefficient in each individual LASSO model. The R package glmnet (v2.0-13) was used.

The most predictive parameters identified by the LASSO model were then used to train a random forest classifier (RFC) on the discovery cohort. For the RFC, 75% of the data from the discovery cohort was used as a training set for model training and 25% of data from the discovery cohort was used for testing. The CD274 (PD-L1 only) model was used as a baseline model to compare predictive performance of the biomarker signature. The classifier was trained as a binary outcome model and the following combinations of outcome classes were used: (ED+PD) vs (PR+CR), (ED+PD+PR) vs CR, (ED+PD) vs CR, ED vs CR. By using a range of binary outcomes it was possible to investigate the specific impact of individual markers for each RECIST 1.1 status category and evaluate model performance sensitivity to the class definition itself.

The trained model with the top performing biomarkers was then validated on a validation cohort - NCT04860076 study data. As mentioned above, the procedure of model training was totally blind with respect to the validation cohort: the model was trained on 75% of the discovery cohort (dbGaP) and then applied "as-is" to make prediction calls on the NCT04860076 data (validation dataset). The predicted calls were then compared with the ground-truth annotation obtained from the hospitals. Model performance during model training, testing and validation was always evaluated using ROC AUC metrics.

### Bioinformatics analysis

After counting reads mapped to the exons of annotated genes (Ensembl release 100 [doi: 10.1093/nar/gkaa942]), the gene hit counts table was used for downstream differential gene expression analysis. Using DESeq2 (v 1.26.0) [doi: 10.1186/s13059-014-0550-8] R package, a comparison of gene expression between the customer-defined groups of samples was performed. The Wald test was used to generate p-values, and log2 fold change values were calculated. P-values were corrected for multiple testing using Benjamini-Hochberg method. Genes with an adjusted p-value < 0.05 were called as differentially expressed genes for each comparison.

Functional enrichment analysis was performed on the differentially expressed set of genes by implementing Over Representation Analysis (ORA) from clusterProfiler (v 3.16.1) [doi: 10.1016/j.xinn.2021.100141] R package. Functional annotation was performed against Gene Ontology [doi: 10.1038/75556, 10.1093/nar/gkaa1113] and KEGG [doi: 10.1093/nar/28.1.271 databases.

Boxplots and violin plots were used to visualize log2-transformed normalized gene expression in groups. Scaled log2-transformed normalized expression of the predicted important sets of genes was visualized with heatmap.

### Statistics and reproducibility.

Unless otherwise stated, all two-group comparisons for continuous variables use the two-sided Student's t-test (R function t.test). Unless otherwise stated, FDR-corrected p values are reported.

### RESULTS

### Study design, patient cohorts, and response assessment.

Across cohorts, 15% of patients were treated with combinations of PD1/L1 with CTLA4, and 85% with PD1/L1 as monotherapy (Table 1). The distribution of patients expressing a specific type of treatment response (RECIST) was comparable between discovery and validation sets (Table 2), except for the PR category (partial response). Discovery dataset contained nearly 3x more PR patients compared with the validation cohort. For other RECIST 1.1 categories patient distributions were comparable.

**Table 1. Patient distribution by treatment type in discovery and validation cohorts**

| | **Ipilimumab + Nivolumab** | **Ipilimumab + Pembrolizumab** | **Nivolumab** | **Pembrolizumab** |
|---|---|---|---|---|
| dbGaP (discovery cohort) | 7 | 25 | 91 | 87 |
| NCT04860076 (validation cohort) | 10 | 0 | 43 | 9 |

**Table 2. Patient distribution by RECIST 1.1. category in discovery and validation cohorts; ED - early death; PD - progressive disease; SD - stable disease; PR - partial response; CR - complete response.**

| | **dbGaP (discovery cohort)** | | **UZA (validation cohort)** | |
|---|---|---|---|---|
| | **Patients** | **%** | **Patients** | **%** |
| **ED** | 33 | 15.7 | 13 | 20.6 |
| **PD** | 58 | 27.6 | 24 | 38.1 |
| **CR** | 34 | 16.2 | 6 | 9.5 |
| **PR** | 54 | 25.7 | 6 | 9.5 |
| **CR** | 31 | 14.8 | 14 | 22.2 |
| **Total:** | 176 | 100% | 57 | 100% |

### RNA extraction and RNA quality.

Overall, most of the RNA samples from the NCT04860076 clinical study passed quality thresholds (DV200 > 30%), and the amount of extracted RNA was sufficient for downstream whole transcriptome sequencing. However we observed a high level of DNA contamination which could not be successfully eliminated with single DNAse treatment. The remaining DNA contamination caused higher intronic and intergenic read coverage than expected. Nevertheless, adequate exonic read coverage that represents transcript abundance was achieved.

### RNA-seq data processing, normalization and PCA analyses.

The PCA-based inspection of the data distributions between the discovery and validation cohort revealed considerable variability along the PC1 axis between different studies and partial variability along the PC2 axis (Figure 1A). Post-combat normalized values allowed to considerably reduce study-specific differences, and achieve acceptable levels of normalization between different datasets (Figure 1B). Furthermore, the statistical inspection of the selected biomarkers (see Results below) confirmed a good level of concordance between the distribution of log2 normalized expression values between the discovery and validation cohorts (Figure 2).

### Predictive signature discovery and validation using machine learning.

LASSO-based learner identified the signature of 25 genes from which 10 genes (TIGIT, PIK3CD, IKZF3, ITGAL, CD247, LAX1, CD3G, CD3E, IGHV1.3, IGHV4.4) accounted for the majority of explanatory power (Table 3) out of which further 3 genes (CD247, LAX1 and IKZF3) were the most predictive in the machine learning models. These markers displayed the highest discriminatory power irrespective of the types of statistical analyses and between-group comparison we undertook (Figure 3A and 3B). Importantly, the markers were identified solely by the discovery cohort and then were explored for confirmation on the validation cohort (Figure 3C and 3D).

CRF model performance on discovery (testing set) and validation set allowed to achieve a high discriminatory performance between RECIST 1.1. categories (Table 4) and depending on the classification ranged from 73% to 78% on validation cohort for a full set of 25 markers (Figures 4A) and 64% to 77% on validation cohort for a 3-marker model (Figure 4B). In all cases the 3-marker model (CD247, LAX1, and IKZF3) and complete model (25 biomarkers) outperformed the baseline model with CD274 (PD-L1 inhibitor only) marker (Figure 4C). The biggest predictive increase in ROC AUC (+15%) was achieved with a 3-marker model (CD247, LAX1, and IKZF3) and an addition of 22 more biomarkers (CD3G, CD3E, CXCR6, SLAMF7, MYO1G, TIGIT, ICOS, CLEC4C, CXCR3, TLR8, ITGAL, FCGR2A, IDO1, TRAF3IP3, PIKCD, IGLV1.36, TRBJ2.7, TRAV14DV4, TRAJ16, IGH1.3, IGH4.28, IGHV4.31, IGHV4.4) led to further model improvement of ~5% ROC AUC.

**Table 3. Summary of the variable importance across different binary classifications with CRF model on validation cohort. Top 10 markers for each classification is shown; markers are based on the 100CV runs of CRF.**

| **ED vs CR** | **ED+PD vs CR** | **ED+PD vs PR+CR** | **ED+PD+PR vs CR** |
|---|---|---|---|
| IGHV1.3 | IKZF3 | PIK3CD | IGHV4.4 |
| CD247 | CD3G | LAX1 | LAX1 |
| TIGIT | CD247 | CXCR6 | IGHV1.3 |
| CD3G | TIGIT | SLAMF7 | CD3G |
| IKZF3 | CD3E | CD247 | CD247 |
| CD3E | PIK3CD | ITGAL | TRAF3IP3 |
| TLR8 | ITGAL | TIGIT | CD3E |
| PIK3CD | CXCR3 | MYO1G | ITGAL |
| CLEC4C | FCGR2A | CD3G | IDO1 |
| FCGR2A | IGHV1.3 | CD3E | IGHV4.28 |

**Table 4. ROC AUC performance scores obtained by applying a CRF model trained on the discovery cohort (training set) to the validation cohort; comparison is given between 25-biomarkers signature model, 3 top biomarkers signature and CD274 (baseline, PD-L1 only) models.**

| | Validation Cohort, 25-biomarkers based model | Validation Cohort, 3 biomarkers based model | Validation Cohort, CD274 (PD-L1 inhibitor only) baseline model |
|---|---|---|---|
| ED vs CR | 73% | 69% | 59% |
| ED+PD vs CR | 78% | 67% | 52% |
| ED+PD vs PR+PR | 72% | 77% | 59% |
| ED+PD+PR vs CR | 75% | 64% | 53% |
| **Overall performance:** | **75%** | **70%** | **56%** |

### Bioinformatics analysis

The differential expression analyses revealed that the majority of the DEG genes are downregulated in the worse-responding subgroups. The top biomarkers identified by the LASSO-learner ML model predominantly passed the FDR-corrected significance threshold of 0.05 and were supported on both discovery and validation cohorts (Figure 5A and 5B). All of the markers are downregulated in the ED, PD, SD subgroups compared to PR and CR subgroups.

The heatmap of the biomarker panel showed a reasonable clustering of the patients with respect to RECICT groups in the discovery cohort (Figure 6A) and even more consistent clustering for the validation cohort (Figure 6B).

### REFERENCES

Davis and Patel 2019. "The role of PD-L1 expression as a predictive biomarker: an analysis of all US Food and Drug Administration (FDA) approvals of immune checkpoint inhibitors". J. Immunother. Cancer 7(1): 278.
Eisenhauer et al., 2009. "New response evaluation criteria in solid tumours : Revised RECIST guideline (version 1.1)" European Journal of Cancer; 45 (228-247).
Ferrara et al. 2020a. "Single or combine immune checkpoint inhibitors compared to first-line platinum-based chemotherapy with or without bevacizumab for people with advanced non-small cell lung cancer". Cochrane Database Syst Rev; 12.
Ferrara et al. 2020b. "Comparison of Fast-Progression, Hyperprogressive Disease, Early Deaths in Advanced Non-Small-Cell Lung Cancer treated with PD-1/PD-L1 inhibitors or chemotherapy". JCO Precision Oncology 4 ; 829-840.

## Claims

1. A method for determining and/or predicting the response or resistance to treatment with an immunotherapeutic agent that is an immune checkpoint inhibitor or a combination of immune checkpoint inhibitors in a subject diagnosed with cancer, said method comprising analyzing the expression level of the biomarkers CD247, LAX1, and IKZF3 in a sample of the subject wherein the sample contains or is suspected to contain tumor cells.

2. The method of claim 1, wherein the immunotherapeutic agent is selected from the group consisting of a PD-1 targeting agent, a PD-L1 targeting agent, a CTLA-1 targeting agent, and a combination thereof.

3. The method according to claims 1 or 2 wherein the sample is a tumor tissue sample or a fluid sample comprising tumor cells; preferably wherein the sample is a tumor tissue sample or a blood sample comprising circulating tumor cells.

4. The method of any one of claims 1 to 3 further comprising analyzing the expression level of the biomarkers CD3G and ITGAL in the sample of the subject.

5. The method according to any of the preceding claims wherein the expression level of at least 2, preferably at least 4, even more preferably all of the biomarkers CD3E, TIGIT, PIK3CD, IGHV1.3, and IGHV4.4 is analyzed in a sample of the subject.

6. The method according to any of the preceding claims, wherein the expression level of the biomarkers is compared to a corresponding reference value or threshold value that is characteristic of a subject with a known response to treatment with the immunotherapeutic agent.

7. The method according to any of the preceding claims wherein on the basis of the expression level of the biomarkers a risk score is obtained, said risk score representing the likelihood for the response to the immunotherapeutic agent.

8. The method according to claim 7, wherein the risk score is obtained and calculated using a pre-trained machine learning model and the expression level of the biomarkers as input values.

9. The method according to any of the preceding claims wherein the response is selected from the RECIST 1.1 response criteria.

10. Use of a kit comprising:
- means for measuring the expression level of the biomarkers CD247, LAX1, and IKZF3 in a sample of the subject;
- optionally, means for measuring the expression level of the biomarkers CD3G and ITGAL in a sample of the subject;
- optionally, means for measuring the expression level of the biomarkers CD3E, TIGIT, PIK3CD, IGHV1.3, IGHV4.4 in a sample of the subject;
- optionally, a reference value or threshold value for the biomarkers CD247, LAX1, and IKZF3;
- optionally, a reference value or threshold value for the biomarkers CD3G and ITGAL ;
- optionally, a reference value or threshold value for the biomarkers CD3E, TIGIT, PIK3CD, IGHV1.3, IGHV4.4,
in the method according to any one of the claims 1 to 9.

11. A computer-implemented method for monitoring, predicting or determining the response of a subject diagnosed with cancer to treatment with an immunotherapeutic agent that is an immune checkpoint inhibitor or a combination of immune checkpoint inhibitors, the method comprising:
(a) providing quantified expression levels of the biomarkers CD247, LAX1, and IKZF3 in a sample of the subject wherein the sample contains or is suspected to contain tumor cells;
(b) optionally providing quantified expression levels of the biomarkers CD3G and ITGAL in a sample of the subject wherein the sample contains or is suspected to contain tumor cells;
(c) normalizing the quantified expression levels whereby normalization occurs via comparison with data obtained from corresponding assessments and expression levels from a reference set;
(d) classifying whether the normalized values of step (c) exceed a predetermined threshold;
(e) obtaining a risk score of said normalized values, wherein said risk score is calculated using a pre-trained machine learning model, and wherein the risk score represents the likelihood for the response to the immunotherapeutic agent by the subject.

12. The method according to claim 11 wherein the immunotherapeutic agent is selected from the group consisting of a PD-1 targeting agent, a PD-L1 targeting agent, a CTLA-4 targeting agent, and a combination thereof.

13. The method according to claim 11 or 12 wherein the sample is a tumor tissue sample or a fluid sample comprising tumor cells; preferably wherein the sample is a tumor tissue sample or a blood sample comprising circulating tumor cells.

14. The method according to any of the preceding claims 1 to 9, or 11 to 13, or the use of claim 10 wherein the biomarker is a protein coding gene, in particular wherein the expression level of the biomarker is the RNA-based expression level of the protein coding gene.

15. The method according to any of the preceding claims 1 to 9, or 11 to 14, or the use of claim 10 or 14 wherein the subject is diagnosed with melanoma; preferably diagnosed with stage II, stage III or stage IV melanoma.

## Patentansprüche

1. Verfahren zum Bestimmen und/oder Vorhersagen des Ansprechens auf oder der Resistenz gegen eine Behandlung mit einem Immuntherapeutikum, das ein Immun-Checkpoint-Inhibitor oder eine Kombination von Immun-Checkpoint-Inhibitoren ist, bei einem Individuum, bei dem Krebs diagnostiziert wurde, wobei das Verfahren das Analysieren des Expressionsspiegels der Biomarker CD247, LAX1 und IKZF3 in einer Probe des Individuums umfasst, wobei die Probe Tumorzellen enthält oder vermutlich enthält.

2. Verfahren nach Anspruch 1, wobei das Immuntherapeutikum aus der aus einem PD-1 ansteuernden Agens, einem PD-L1 ansteuernden Agens, einem CTLA-1 ansteuernden Agens und einer Kombination davon bestehenden Gruppe ausgewählt ist.

3. Verfahren nach Anspruch 1 oder 2, wobei die Probe eine Tumorgewebeprobe oder eine Fluidprobe ist, die Tumorzellen umfasst, wobei vorzugsweise die Probe eine Tumorgewebeprobe oder eine Blutprobe ist, die zirkulierende Tumorzellen umfasst.

4. Verfahren nach einem der Ansprüche 1 bis 3, das ferner Analysieren des Expressionsspiegels der Biomarker CD3G und ITGAL in der Probe des Individuums umfasst.

5. Verfahren nach einem der vorhergehenden Ansprüche, wobei der Expressionsspiegel von mindestens 2, vorzugsweise mindestens 4, noch stärker bevorzugt allen der Biomarker CD3E, TIGIT, PIK3CD, IGHV1.3 und IGHV4.4 in einer Probe des Individuums analysiert wird.

6. Verfahren nach einem der vorhergehenden Ansprüche, wobei der Expressionsspiegel der Biomarker mit einem entsprechenden Referenzwert oder Schwellenwert verglichen wird, der für ein Individuum mit bekanntem Ansprechen auf eine Behandlung mit dem Immuntherapeutikum charakteristisch ist.

7. Verfahren nach einem der vorhergehenden Ansprüche, wobei auf der Basis des Expressionsspiegels der Biomarker ein Risikowert erhalten wird, wobei der Risikowert die Wahrscheinlichkeit für das Ansprechen auf das Immuntherapeutikum darstellt.

8. Verfahren nach Anspruch 7, wobei der Risikowert unter Verwendung eines vortrainierten Maschinenlernmodells und des Expressionsspiegels der Biomarker als Eingabewerte erhalten und berechnet wird.

9. Verfahren nach einem der vorhergehenden Ansprüche, wobei das Ansprechen aus den RECIST 1,1-Kriterien für das Ansprechen ausgewählt wird.

10. Verwendung eines Kits, das Folgendes umfasst:
- Mittel zum Messen des Expressionsspiegels der Biomarker CD247, LAX1 und IKZF3 in einer Probe des Individuums,
- gegebenenfalls Mittel zum Messen des Expressionsspiegels der Biomarker CD3G und ITGAL in einer Probe des Individuums,
- gegebenenfalls Mittel zum Messen des Expressionsspiegels der Biomarker CD3E, TIGIT, PIK3CD, IGHV1.3, IGHV4.4 in einer Probe des Individuums,
- gegebenenfalls einen Referenzwert oder Schwellenwert für die Biomarker CD247, LAX1 und IKZF3,
- gegebenenfalls einen Referenzwert oder Schwellenwert für die Biomarker CD3G und ITGAL,
- gegebenenfalls einen Referenzwert oder Schwellenwert für die Biomarker CD3E, TIGIT, PIK3CD, IGHV1.3, IGHV4.4,
bei dem Verfahren nach einem der Ansprüche 1 bis 9.

11. Computerimplementiertes Verfahren zum Überwachen, Vorhersagen oder Bestimmen des Ansprechens eines Individuums, bei dem Krebs diagnostiziert wurde, auf eine Behandlung mit einem Immuntherapeutikum, das ein Immun-Checkpoint-Inhibitor oder eine Kombination von Immun-Checkpoint-Inhibitoren ist, wobei das Verfahren Folgendes umfasst:
(a) Bereitstellen quantifizierter Expressionsspiegel der Biomarker CD247, LAX1 und IKZF3 in einer Probe des Individuums, wobei die Probe Tumorzellen enthält oder vermutlich enthält,
(b) gegebenenfalls Bereitstellen quantifizierter Expressionsspiegel der Biomarker CD3G und ITGAL in einer Probe des Individuums, wobei die Probe Tumorzellen enthält oder vermutlich enthält,
(c) Normalisieren der quantifizierten Expressionsspiegel, wobei die Normalisierung mittels Vergleich mit Daten erfolgt, die aus entsprechenden Feststellungen und Expressionsspiegeln aus einem Referenzsatz erhalten werden,
(d) Klassifizieren, ob die normalisierten Werte von Schritt (c) eine zuvor festgelegte Schwelle überschreiten,
(e) Erhalten eines Risikowerts aus den normalisierten Werten, wobei der Risikowert unter Verwendung eines vortrainierten Maschinenlernmodells berechnet wird und wobei der Risikowert die Wahrscheinlichkeit eines Ansprechens auf das Immuntherapeutikum durch das Individuum darstellt.

12. Verfahren nach Anspruch 11, wobei das Immuntherapeutikum aus der aus einem PD-1 ansteuernden Agens, einem PD-L1 ansteuernden Agens, einem CTLA-4 ansteuernden Agens und einer Kombination davon bestehenden Gruppe ausgewählt ist.

13. Verfahren nach Anspruch 11 oder 12, wobei die Probe eine Tumorgewebeprobe oder eine Fluidprobe ist, die Tumorzellen umfasst, wobei vorzugsweise die Probe eine Tumorgewebeprobe oder eine Blutprobe ist, die zirkulierende Tumorzellen umfasst.

14. Verfahren nach einem der vorhergehenden Ansprüche 1 bis 9 oder 11 bis 13 oder Verwendung nach Anspruch 10, wobei der Biomarker ein Protein-codierendes Gen ist, wobei insbesondere der Expressionsspiegel des Biomarkers der auf RNA basierende Expressionsspiegel des Proteincodierenden Gens ist.

15. Verfahren nach einem der vorhergehenden Ansprüche 1 bis 9 oder 11 bis 14 oder Verwendung nach Anspruch 10 oder 14, wobei bei dem Individuum ein Melanom, vorzugsweise ein Melanom des Stadiums II, Stadiums III oder Stadiums IV, diagnostiziert wird.

## Revendications

1. Procédé de détermination et/ou de prédiction de la réponse ou de la résistance à un traitement par un agent immunothérapeutique qui est un inhibiteur de point de contrôle immunitaire ou une combinaison d'inhibiteurs de point de contrôle immunitaire, chez un sujet chez qui un cancer a été diagnostiqué, ledit procédé comprenant l'analyse du taux d'expression des biomarqueurs CD247, LAX1 et IKZF3 dans un échantillon du sujet, l'échantillon contenant ou étant suspecté de contenir des cellules tumorales.

2. Procédé selon la revendication 1, dans lequel l'agent immunothérapeutique est choisi dans le groupe constitué par un agent ciblant PD-1, un agent ciblant PD-L1, un agent ciblant CTLA-1, et une combinaison de ceux-ci.

3. Procédé selon la revendication 1 ou 2, dans lequel l'échantillon est un échantillon de tissu tumoral ou un échantillon de fluide comprenant des cellules tumorales ; de préférence, l'échantillon étant un échantillon de tissu tumoral ou un échantillon de sang comprenant des cellules tumorales circulantes.

4. Procédé selon l'une quelconque des revendications 1 à 3, comprenant en outre l'analyse du taux d'expression des biomarqueurs CD3G et ITGAL dans l'échantillon du sujet.

5. Procédé selon l'une quelconque des revendications précédentes, dans lequel le taux d'expression d'au moins 2, de préférence au moins 4, et plus particulièrement de l'ensemble des biomarqueurs CD3E, TIGIT, PIK3CD, IGHV1.3 et IGHV4.4 est analysé dans un échantillon du sujet.

6. Procédé selon l'une quelconque des revendications précédentes, dans lequel le taux d'expression des biomarqueurs est comparé à une valeur de référence ou à une valeur seuil correspondante, caractéristique d'un sujet présentant une réponse connue au traitement par l'agent immunothérapeutique.

7. Procédé selon l'une quelconque des revendications précédentes, dans lequel, sur la base du taux d'expression des biomarqueurs, un score de risque est obtenu, ledit score de risque représentant la probabilité de réponse à l'agent immunothérapeutique.

8. Procédé selon la revendication 7, dans lequel le score de risque est obtenu et calculé à l'aide d'un modèle d'apprentissage automatique préentraîné, dans lequel les taux d'expression des biomarqueurs sont des valeurs d'entrée.

9. Procédé selon l'une quelconque des revendications précédentes, dans lequel la réponse est sélectionnée à partir des critères de réponse RECIST 1.1.

10. Utilisation d'un kit comprenant :
- des moyens pour mesurer le taux d'expression des biomarqueurs CD247, LAX1 et IKZF3 dans un échantillon du sujet ;
- facultativement, des moyens pour mesurer le taux d'expression des biomarqueurs CD3G et ITGAL dans un échantillon du sujet ;
- facultativement, des moyens pour mesurer le taux d'expression des biomarqueurs CD3E, TIGIT, PIK3CD, IGHV1.3, IGHV4.4 dans un échantillon du sujet ;
- facultativement, une valeur de référence ou une valeur seuil pour les biomarqueurs CD247, LAX1 et IKZF3 ;
- facultativement, une valeur de référence ou une valeur seuil pour les biomarqueurs CD3G et ITGAL ;
- facultativement, une valeur de référence ou une valeur seuil pour les biomarqueurs CD3E, TIGIT, PIK3CD, IGHV1.3, IGHV4.4,
dans le procédé selon l'une quelconque des revendications 1 à 9.

11. Procédé mise en œuvre par ordinateur de surveillance, de prédiction ou de détermination de la réponse d'un sujet chez qui un cancer a été diagnostiqué à un traitement par un agent immunothérapeutique qui est un inhibiteur de point de contrôle immunitaire ou une combinaison d'inhibiteurs de point de contrôle immunitaire, le procédé comprenant :
(a) la fourniture de taux d'expression quantifiés des biomarqueurs CD247, LAX1 et IKZF3 dans un échantillon du sujet, l'échantillon contenant ou étant suspecté de contenir des cellules tumorales ;
(b) facultativement, la fourniture de taux d'expression quantifiés des biomarqueurs CD3G et ITGAL dans un échantillon du sujet, l'échantillon contenant ou étant suspecté de contenir des cellules tumorales ;
(c) la normalisation des taux d'expression quantifiés, la normalisation étant effectuée par comparaison à des données obtenues à partir d'évaluations correspondantes et des taux d'expression d'un ensemble de référence ;
(d) la classification selon que les valeurs normalisées de l'étape (c) dépassent ou non un seuil prédéterminé ;
(e) l'obtention d'un score de risque desdites valeurs normalisées, ledit score de risque étant calculé à l'aide d'un modèle d'apprentissage automatique préentraîné et le score de risque représentant la probabilité de réponse du sujet à l'agent immunothérapeutique.

12. Procédé selon la revendication 11, dans lequel l'agent immunothérapeutique est choisi dans le groupe constitué par un agent ciblant PD-1, un agent ciblant PD-L1, un agent ciblant CTLA-4, et une combinaison de ceux-ci.

13. Procédé selon la revendication 11 ou 12, dans lequel l'échantillon est un échantillon de tissu tumoral ou un échantillon de fluide comprenant des cellules tumorales ; de préférence, l'échantillon étant un échantillon de tissu tumoral ou un échantillon de sang comprenant des cellules tumorales circulantes.

14. Procédé selon l'une quelconque des revendications précédentes 1 à 9 ou 11 à 13, ou l'utilisation selon la revendication 10, le biomarqueur étant un gène codant pour une protéine, en particulier, le taux d'expression du biomarqueur étant le taux d'expression basé sur l'ARN du gène codant pour une protéine.

15. Procédé selon l'une quelconque des revendications précédentes 1 à 9 ou 11 à 14, ou l'utilisation selon la revendication 10 ou 14, le sujet étant un sujet chez qui un mélanome a été diagnostiqué ; de préférence, chez qui un mélanome de stade II, III ou IV a été diagnostiqué.
